# EUROPÄISCHE PATENTSCHRIFT

(11) **EP 0 004 648 B1**
(45) Veröffentlichungstag und Bekanntmachung des Hinweises auf die Patenterteilung: **25.08.1982**
(21) Anmeldenummer: 79100962.4
(22) Anmeldetag: 30.03.1979
(51) Int. Cl.: C07D 401/04, C07D 409/04, A61K 31/415, A61K 31/44

(54) **Neue Mercaptoimidazolderivate, Verfahren zu deren Herstellung, Mercaptoimidazolderivate zur Behandlung entzündlicher Erkrankungen und diese enthaltende pharmazeutische Präparate**
Mercapto-imidazole derivatives, their preparation, mercapto-imidazole derivatives for the treatment of inflammatory diseases and their pharmaceutical compositions
Dérivés de mercapto-imidazole, procédé pour leur préparation, dérivés de mercapto-imidazole pour le traitement de maladies inflammatoires et leurs compositions pharmaceutiques

(30) Priorität: 11.04.1978 LU 79412
(43) Veröffentlichungstag der Anmeldung: 17.10.1979
(73) Patentinhaber: CIBA-GEIGY AG, 4002 Basel (CH)
(72) Erfinder: Ferrini, Pier Giorgio, Dr., CH-4102 Binningen (CH); Göschke, Richard, Dr., CH-4103 Bottmingen (CH)

## Beschreibung

Die vorliegende Erfindung betrifft substituierte 2-Mercaptoimidazole der Formel 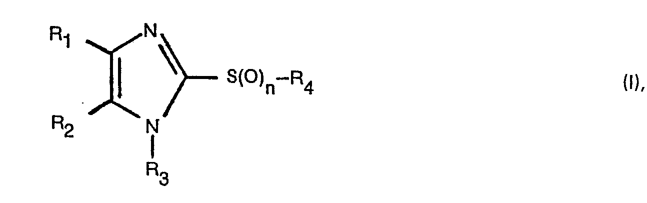 worin mindestens einer der Reste R₁ und R₂ eine unsubstituierte oder durch Niederalkyl, Niederalkoxy, Halogen, Amino, Niederalkylamino, Diniederalkylamino, Alkylen- bzw. 3-Aza-, 3-Oxa- oder 3-Thia- alkylenamino mit jeweils 5 oder 6 Ringgliedern und/oder Trifluormethyl C-substituierte und/oder durch Niederalkyl, Niederalkoxyniederalkyl, Hydroxyniederalkyl oder Oxy N-substituierte monocyclische, über ein C-Atom gebundene, ein Sauerstoff- oder Schwefelatom, ein Schwefel- und ein Stickstoffatom oder mindestens 2 Stickstoffatome aufweisende 5-gliedrige oder mindestens ein Stickstoffatom aufweisende 6-gliedrige Heteroarylgruppe und ein davon verschiedener Rest R₁ oder R₂ eine unsubstituierte oder durch Niederalkyl, Niederalkoxy, Halogen, Amino, Niederalkylamino, Diniederalkylamino, Alkylenamino bzw. 3-Aza-, 3-Oxa- oder 3-Thia-alkylenamino mit jeweils 5 oder 6 Ringgliedern, Nitro und/oder Trifluormethyl substituierte Phenylgruppe bedeutet, R₃ Wasserstoff oder Niederalkyl darstellt, n für 0, 1 oder 2 steht, und R₄ einen unsubstituierten oder durch unsubstituiertes oder durch Niederalkyl, Niederalkoxy, Halogen, Amino, Niederalkylamino, Diniederalkylamino, Alkylenamino, bzw. 3-Aza-, 3-Oxa- oder 3-Thia-alkylenamino mit jeweils 5 oder 6 Ringgliedern, Nitro und/oder Trifluormethyl substituiertes Phenyl substituierten Niederalkyl-, Niederalkenyl- oder Niederalkinylrest oder einen in höherer Stellung als der a-Stellung durch Hydroxy, Niederalkoxy, Niederalkylthio, eine unsubstituierte oder durch Niederalkyl, Niederalkoxy, Halogen, Amino, Niederalkylamino, Diniederalkylamino, Alkylenamino bzw. 3-Aza-, 3-Oxa- oder 3-Thia-alkylenamino mit jeweils 5 oder 6 Ringgliedern, Nitro und/oder Trifluormethyl substituierte Phenoxy- oder Phenylthiogruppe substituierten Niederalkylrest bedeutet, mit der Massgabe, dass Heteroarylreste R₁ und/oder R₂ von ein Schwefelatom aufweisenden 5-gliedrigen und ein Stickstoffatom aufweisenden 6-gliedrigen Heteroarylresten oder Reste R₃ von Wasserstoff verschieden sind, wenn R₄ einen in 2-, 3- oder 4-Stellung durch eine Hydroxygruppe substituierten Niederalkylrest bedeuten, und mit der weiteren Massgabe, dass in Verbindungen der Formel I, worin n für 1 oder 2 steht, Heteroarylreste R₁ und/oder R₂ von ein Schwefel- oder Sauerstoffatom aufweisenden 5-gliedrigen oder ein Stickstoffatom aufweisenden 6-gliedrigen Heteroarylreste verschieden sind, wenn R₄ durch ein zwei Hydroxygruppen, eine Niederalkoxygruppe oder durch gegebenenfalls wie angegeben Phenyl substituiertes Niederalkyl oder unsubstituiertes Niederalkinyl darstellt, und pharmazeutisch verwendbare Salze, vor allem pharmazeutisch verwendbare Säureadditionssalze davon.

Vor und nachstehend sind unter "niederen" organischen Resten und Verbindungen solche zu verstehen, die bis und mit 7, vor allem bis und mit 4, Kohlenstoffatome aufweisen.

Ein Sauerstoff- oder Schwefelatom ein Schwefel- und ein Stickstoffatom oder mindestens zwei Stickstoffatome aufweisende 5-gliedrige oder mindestens ein Stickstoffatom aufweisende 6-gliedrige Heteroarylreste sind über ein C-Atom gebunden. Als Beispiele sind insbesondere Furyl, z.B. 2-Furyl, Thienyl, z.B. 2-Thienyl, Thiazolyl, z.B. 1,3-Thiazolyl-(2) oder-(4), Imidazolyl, z.B. Imidazolyl-(2) oder -(4), Triazolyl, z.B. 1 H- oder 2H-1,2,3-Triazolyl-(4) oder -(5) oder 1 H- oder 2H-1,2,4-Triazolyl-(3) oder -(5), Tetrazolyl, z.B. H-Tetrazolyl-(5), Pyridyl, z.B. 2-, 3- oder 4-Pyridyl, Pyrimidinyl, z.B. 2- oder 4-Pyrimidinyl, oder Triazinyl, z.B. 1,3,5-Triazinyl-(2) oder 1,2,4-Triazinyl-(3) zu nennen.

Als Beispiele für Niederalkylamino, Diniederalkylamino und Alkylenamino bzw. 3-Aza-, 3-Oxa-oder 3-Thiaalkylenamino mit jeweils 5 oder 6 Ringgliedern seien neben Methylamino und Aethylamino vor allem Dimethylamino, Diäthylamino, Pyrrolidino, Piperidino, Morpholino, Thiomorpholino, Piperazino und N'-Niederalkyl-, z.B. N'-Methylpiperazino genannt.

Gegebenenfalls durch Phenyl substituiertes Niederalkyl, Niederalkenyl bzw. Niederalkinyl ist beispielsweise Niederalkyl mit 1 bis 4 C-Atomen, Phenylniederalkyl, wie 1-oder 2-Phenylniederalkyl mit 7 bis 10 C-Atomen, z.B. Benzyl oder 2-Phenyläthyl, Niederalkenyl mit 2 bis 4 C-Atomen, z.B. Vinyl, Allyl oder Methallyl, Phenylniederalkenyl mit 8 bis 10 C-Atomen, z.B. Styryl, Niederalkinyl mit 2 bis 4 C-Atomen, z.B. Aethinyl oder Propargyl, oder Phenylniederalkinyl mit 8 bis 10 C-Atomen, z.B. Phenyläthinyl.

In höherer als der a-Stellung durch Hydroxy substituiertes Niederalkyl (Hydroxyniederalkyl) ist beispielsweise Mono- oder Dihydroxyniederalkyl, hat insbesondere 2 bis 4 C-Atome und ist z.B. 2-Hydroxyäthyl, 2- oder 3-Hydroxypropyl oder 2,3-Dihydroxypropyl.

In höherer als der a-Stellung deren Niederalkoxy substituiertes Niederalkyl (Niederalkoxyniederalkyl) hat beispielsweise je 1 bis 4 C-Atom und ist z.B. 2-Methoxyäthyl, 2-Aethoxyäthyl oder 2-oder 3-Methoxypropyl.

In höher als der a-Stellung durch Niederalkylthio substituiertes Niederalkyl (Niederalkylthioniederalkyl) mit 1 bis 7 C-Atome ist z.B. 2-Methylthioäthyl.

In höherer als der a-Stellung durch gegegebenenfalls wie angegeben substituiertes Phenoxy bzw. Phenylthio substituiertes Niederalkyl mit 1 bis 7 C-Atome im Alkylteil ist z.B. 2-Phenoxyäthyl bzw. 2-Phenylthioäthyl.

Niederalkyl mit 1 bis 7 C-Atome ist z.B. Methyl, Aethyl, Propyl, Isopropyl oder n-, iso-, sek- oder tert.-Butyl oder in zweiter Linie eine der isomeren Pentyl-, Hexyl- oder Heptylgruppen.

Niederalkoxy mit 1 bis 7 C-Atome ist z.B. Methoxy, Aethoxy, Propoxy, Isopropoxy, n-, sek-, iso-oder tert.-Butoxy oder in zweiter Linie eine der isomeren Pentyloxy-, Hexyloxy- oder Heptyloxygruppen.

Niederalkenyl und Niederalkinyl haben beispielsweise 2 bis 4 C-Atome; Niederalkenyl ist z.B. Vinyl, Allyl oder Methallyl, während Niederalkinyl vor allem Aethinyl oder Propargyl bedeutet.

Halogen ist vorzugsweise Halogen bis und mit Atomnummer 35, wie Fluor, Chlor oder Brom.

Salze von Verbindungen der Formel sind vor allem pharmazeutisch verwendbare Säureadditionssalze mit starken Säuren, wie Mineralsäure, z.B. Salze mit Halogenwasserstoffsäuren, vor allem Chlor- oder Bromwasserstoffsäure, d.h. Hydrohalogenide, vor allem Hydrochloride und Hydrobromide, oder Schwefelsäuresalze, d.h. Hydrogensulfate und Sulfate.

Die Erfindung betrifft vor allem substituierte 2-Mercaptoimidazole der Formel I, worin mindestens einer der Reste R₁ und R₂ eine unsubstituierte oder durch Niederalkyl, Niederalkoxy, Halogen, Amino, Niederalkylamino, Diniederalkylamino, Alkylen- bzw. 5-Aza-, 3-Oxa- oder 3-Thia-alkylenamino mit jeweils 5 oder 6 Ringgliedern und/oder Trifluoromethyl C-substituierte und/oder durch Niederalkyl, Niederalkoxyniederalkyl oder Hydroxyniederalkyl, N-substituierte monocyclische, über ein C-Atom gebundene, ein Sauerstoff- oder Schwefelatom, ein Schwefel- und ein Stickstoffatom oder mindestens 2 Stickstoffatome aufweisende 5-gliedrige oder mindestens ein Stickstoffatom aufweisende 6-gliedrige Heteroarylgruppe und ein davon verschiedener Rest R₁ oder R₂ eine unsubstituierte oder durch Niederalkyl, Niederalkoxy, Halogen, Amino, Niederalkylamino, Diniederalkylamino, Alkylenamino bzw. 3-Aza-, 3-Oxa- oder 3-Thia-alkylenamino mit jeweils 5 oder 6 Ringgliedern, Nitro und/oder Trifluormethyl substituierte Phenylgruppe bedeutet, R₃ Wasserstoff oder Niederalkyl darstellt, n für 0 steht und R₄ einen unsubstituierten oder durch unsubstituiertes oder durch Niederalkyl, niederalkoxy, Halogen, Amino, Niederalkylamino, Diniederalkylamino, Alkylenamino, bzw. 3-Aza-, 3-Oxa- oder 3-Thia- alkylenamino mit jeweils 5 oder 6 Ringgliedern, Nitro und/oder Trifluormethyl substituiertes Phenyl substituiertes Niederalkyl-, Niederalkenyl- oder Niederalkinylrest oder einen oder in höherer Stellung als der a-Stellung durch Hydroxy, Niederalkoxy, Niederalkylthio, eine unsubstituierte oder durch Niederalkyl, Niederalkoxy, Halogen, Amino, Niederalkylamino, Diniederalkylamino, Alkylenamino bzw. 3-Aza₋, 3-Oxa- oder 3-Thiaalkylenamino mit jeweils 5 oder 6 Ringgliedern, Nitro und/oder Trifluormethyl substituierte Phenoxy- oder Phenylthiogruppe substituierten Niederalkylrest bedeutet, mit der Massgabe, dass Heteroarylreste R₁ und/oder R₂ von ein Schwefelatom aufweisenden 5-gliedrigen und ein Stickstoffatom aufweisenden 6-gliedrigen Heteroarylresten oder Reste R₃ von Wasserstoff verschieden sind, wenn R₄ einen in 2-, 3- oder 4-Stellung durch eine Hydroxygruppe substituierten Niederalkylrest bedeuten, und pharmazeutisch verwendbare Salze, vor allem pharmazeutisch verwendbare Säureadditionssalze davon.

Die Verbindungen der Formel I weisen wertvolle pharmakologische Eigenschaften auf. Insbesondere zeigen sie eine ausgeprägte anti-inftammatorische und/oder antinociceptive Wirksamkeit sowie eine Flemmwirkung auf die Prostaglandinsynthese. So erweisen sie sich an der Ratte im Kaolinp-Jotenoedem nach Helv. Physiol. Acta 25, 156 (1967) im Dosisbereich von etwa 50 bis 200 mg/kg p.o. und an der Maus im durch Phenyl-p-benzochinon ausgelösten Writhing-Syndrom nach J. Pharmacol. exp. Therap. 125, 237 (1959) im Dosisbereich von etwa 100 mg/kg p.o. als ausgezeichnet wirksam.

Ferner hemmen sie in vitro im Konzentrationsbereich von etwa 10 bis 30 ml/1 die Prostaglandinsynthese aus Arachidonsäure, nachgewiesen in der Versuchanordnung nach Prostaglandins 7, 123 (1974).

Aus der DE - A - 2.635.876 waren bereits antinflammatorische wirksame Mercaptoimidazolderivate bekannt, deren 2-Mercaptogruppe durch gegebenenfalls in bestimmter Weise substituiertes Niederalkyl bzw. durch bestimmte Niederalkenylgruppen und deren 4- und 5-Stellung durch gegebenenfalls substituierte Phenylgruppen substituiert sind. Auch waren aus der DE-A-2 805 167 2-Polyhalogenniederalkylthioimidazole mit antiinflammatorischer Wirkung bekannt, deren 4- und 5-Stellung durch mindestens eine Thienyl-, Pyridyl- oder Furylgruppe und gegebenenfalls zusätzlich durch gegebenenfalls substituiertes Phenyl substituiert sind. Strukturell fernerstehende Mercaptoimidazol waren ferner aus der US-A-2.981.739, der US-A-3.714.179 und der FR-A-2.194.433 vorbekannt. Ferner sei auf die ebenfalls teilweise zum Stand der Technik gehörenden, nicht vorpublizierten EP-Anmeldungen Nr. 353, 5545 und 13732 verwiesen.

Den aus der DE - A - 2.805.167 bekannten Mercaptoimidazolderivaten gegenüber zeichnen sich Verbindungen der Formel I durch einen günstigeren therapeutischen Index aus.

Die Verbindungen der Formel sind deshalb vorzüglich geeignet als Wirkstoffe von pharmazeutischen Präparaten zur Behandlung entzündlicher Erkrankungen, vor allem chronischer Entzüdnungen des rheumatischen Formenkreises, wie der chronischen Arthritis.

Die Erfindung betifft dementsprechend ebenfalls Verbindungen der Formel 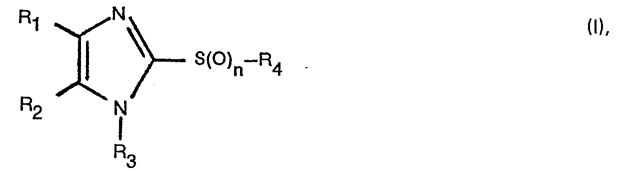 worin mindestens einer der Reste R₁ und R₂ eine unsubstituierte oder durch Niederalkyl, Niederalkoxy, Halogen, Amino, Niederalkylamino, Diniederalkylamino, Alkylen- bzw. 3-Aza-, 3-Oxa- oder 3-Thia- alkylenamino mit jeweils 5 oder 6 Ringgliedern und/oder Trifluormethyl C-substituierte und/oder durch Niederaikyl, Niederalkoxyniederalkyl, Hydroxyniederalkyl oder Oxy N-substituierte monocyclische, über ein C-Atom gebundene, ein Sauerstoff- oder Schwefelatom, ein Schwefel- und ein Stickstoffatom oder mindestens 2-Stickstoffatome aufweisende 5-gliedrige oder mindestens 2-Stickstoffatome aufweisende 5-gliedrige oder mindestens ein Stickstoffatom aufweisende 6 gliedrige Heteroarylgruppe und ein davon verschiedener Rest R₁ oder R₂ eine unsubstituierte oder durch Niederalkyl, Niederalkoxy Halogen, Amino, Niederalkylamino, Diniederalkylamino, Alkylenamino bzw. 3-Aza-, 3-Oxa- oder 3-Thia- alkylenamino mit jeweils 5 oder 6 Ringgliedern, Nitro und/oder Trifluormethyl substituierte Phenylgruppe bedeutet, R₃ Wasserstoff oder Niederalkyl darstellt, n für 0, 1 oder 2 steht, und R₄ einen unsubstituierten oder durch unsubstituiertes oder durch Niederalkyl, Niederalkoxy, Halogen, Amino, Niederalkylamino, Diniederalkylamino, Alkylenamino bzw. 3-Aza-, 3-Oxa- oder 3-Thia-alkylenamino mit jeweils 5 oder 6-Ringgliedern, Nitro und/oder Trifluormethyl substituiertes Phenyl substituierten Niederalkyl-, Niederalkenyl- oder Niederalkinylrest oder einen in höherer als der a-Stellung durch Hydroxy, Niederalkoxy, Niederalkylthio, oder eine unsubstituierte oder durch Niederalkyl, Niederalkoxy, Halogen, Amino, Niederalkylamino, Diniederalkylamino, Alkylenamino bzw. 3-Aza-, 3-Oxa-oder 3-Thia-alkylenamino mit jeweils 5 oder 6 Ringgliedern, Nitro und/oder Trifluormethyl substituierte Phenoxy- oder Phenylthiogruppe substituierten Niederalkylrest bedeutet, mit der Massgabe, dass in Verbindungen der Formel I, worin n für 1 oder 2 steht, Heteroarylreste R₁ und/oder R₂ von ein Schwefel-oder Sauerstoffatom aufweisenden 5-gliedrigen oder ein Stickstoffatom aufweisenden 6-gliedrigen Heteroarylreste verschieden sind, wenn R₄ einen durch ein oder zwei Hydroxygruppen, eine Niederalkoxygruppe oder durch gegebenenfalls wie angegeben substituiertes Phenyl substituierten Niederalkyl- oder unsubstituierten Niederalkinylrest darstellt, und ihre pharmazeutisch verwendbaren Säureadditionssalze zur Anwendung in einem Verfahren zur therapeutischen Behandlung des menschlichen oder tierischen Körpers, diese enthaltende pharmazeutische Präparate und ihre Verwendung des Arzneimittel bzw. zur Herstellung von Arzneimitteln auf nichtchemischen Wege.

Die Erfindung betrifft in erster Linie solche der eingangs definierten Verbindungen, worin in der Formel 1 mindestens einer der Reste R₁ und R₂ Furyl, Thienyl, Thiazolyl, Imidazolyl, Triazolyl, Tetrazolyl, Pyridyl, Pyrimidinyl oder Triazinyl bedeutet, wobei Ringkohlenstoffatome der genannten Reste R₁ und R₂ durch Niederalkyl, Niederalkoxy, Halogen mit und bis Atomnummer 35, Trifluormethyl, Amino, Niederalkylamino und/oder Alkylen- bzw. 3-Aza-, 3-Oxa- oder 3-Thio-alkylenamino mit jeweils 5 oder 6 Ringgliedern, Ringstickstoffatome von Pyrimidinyl-, Triazinyl- oder Pyridylresten ferner durch Oxy substituiert sein können, und ein davon verschiedener Rest R₁ oder R₂ Phenyl darstellt, welches durch Niederalkyl, Niederalkoxy, Halogen mit und bis Atomnummer 35, Trifluormethyl, Nitro, Amino, Niederalkylamino und/oder Alkylen- bzw. 3-Aza-, 3-Oxa- oder 3-Thia-alkylenamino mit jeweils 5 oder 6 Ringgliedern substituiert sein kann, R₃ Wasserstoff oder Niederalkyl bedeutet, n für 0, 1 oder steht, und R₄ einen unsubstituierten oder durch gegebenenfalls durch Niederalkyl, Niederalkoxy, Halogen mit und bis Atomnummer 35, Trifluormethyl, Nitro, Amino, Niederalkylamino und/oder Alkylen- bzw. 3-Aza-, 3-Oxa- oder 3-Thia-alkylenamino mit jeweils 5 oder 6 Ringgliedern substituiertes Phenyl substituierten Niederalkyl-, Niederalkenyl- oder Niederalkinylrest mit nicht mehr als 7 C-Atomen oder einen in höherer als der a-Stellung durch Hydroxy, Niederalkoxy, Niederalkylthio, oder eine gegebenenfalls durch Niederalkyl Niederalkoxy, Halogen mit und bis Atomnummer 35, Trifluormethyl, Nitro, Amino, Niederalkylamino und/oder Alkylen- bzw. 3-Aza-, 3-Oxa- oder 3-Thia-alkylenamino mit jeweils 5 oder 6 Ringgliedern substituierte Phenoxy- oder Phenylthiogruppe substituierten Niederalkylrest mit nicht mehr als 7 C-Atomen bedeutet, und deren, vorzugsweise pharmazeutisch verwendbare, Säureadditionssalze sowie diese Verbindungen enthaltende pharmazeutische Präparate und ihre Verwendung.

Die Erfindung betrifft vor allem Verbindungen der Formel I, worin einer der Reste R₁ und R₂ eine gegebenenfalls substituierte Pyridylgruppe und der andere eine gegebenenfalls substituierte Phenyl-oder Pyridyl- gruppe bedeutet, R₃ Wasserstoff oder Niederalkyl mit 1 bis 4 C-Atomen, wie Methyl darstellt, n für 0 steht, und R₄ gegebenenfalls durch Phenyl oder in einer höheren als der a-Stellung durch Hydroxy, Niederalkoxy oder Niederalkylthio mit 1 bis 4 C-Atomen, Phenoxy oder Phenylthio substituiertes Niederalkyl mit 1 bis 7 C-Atomen oder einen gegebenenfalls durch Phenyl substituierten Niederalkenyl-oder Niederalkinylrest mit 2 bis 4 C-Atomen bedeutet, wobei Pyridyl-, Phenyl-, Phenoxy- oder Phenylthiogruppen durch Niederalkyl mit 1 bis 4 C-Atomen, wie Methyl, Niederalkoxy mit 1 bis 4 C-Atomen, wie Methoxy oder Aethoxy, Halogen bis und mit Atomnummer 35, wie Chlor, Nitro, Amino oder N,N-Diniederalkylamino mit jeweils 1 bis 4 C-Atomen, wie Dimethylamino, substituiert sein können, und deren Salze, sowie diese Verbindungen enthaltende pharmazeutische Präparate und ihre Verwendung.

Die Erfindung betrifft vorzugsweise Verbindungen der Formel I, worin einer der Reste R₁ oder R₂ Pyridyl und der andere Phenyl bedeutet, wobei diese Reste unabhängig voneinander durch Niederalkyl mit 1 bis 4 C-Atomen, wie Methyl, Niederalkoxy mit 1 bis 4 C-Atomen, wie Methoxy oder Aethoxy, Halogen bis und mit Atomnummer 35, wie Chlor, und/oder N,N-Diniederalkylamino mit jeweils 1 bis 4 C-Atomen, wie Dimethylamino, substituiert sein können, vorzugsweise jedoch unsubstituiert sind, R₃ Wasserstoff oder Niederalkyl mit 1 bis 4 C-Atomen, wie Methyl, bedeutet, n für 0 steht, und R₄ Niederalkyl mit 1 bis 4 C-Atomen, wie Methyl, Aethyl, Propyl, Isopropyl oder Butyl, Niederalkenyl mit 2 bis 4 C-Atomen, wie Allyl, gegebenenfalls durch Niederalkyl mit 1 bis 4 C-Atomen, wie Methyl, Niederalkoxy mit 1 bis 4 C-Atomen, wie Methoxy oder Aethoxy, und/oder Halogen bis und mit Atomnummer 35, wie Chlor, substituiertes Phenylniederalkyl mit 1 bis 4 C-Atomen im Alkylteil, die Hydroxygruppe(n) in höherer als α-Stellung tragendes Mono- oder Dihydroxyniederalkyl mit 1 bis 4 C-Atomen, wie 2-Hydroxyäthyl, die Alkoxy- bzw. Alkylthiogruppe in einer höheren als der a-Stellung tragendes Niederalkoxy- oder Niederalkylthio-niederalkyl mit jeweils 1 bis 4 C-Atomen in den Alkylteilen, wie 2-Methoxyäthyl oder 2-Methylthioäthyl, bedeutet, und ihre Säureadditionssalze, sowie diese Verbindungen enthaltende pharmazeutische Präparate und ihre Verwendung.

Die Erfindung betrifft vor allem Verbindungen der Formel 1, worin einer der Reste R₁ und R₂ unsubstituiertes Pyridyl, wie 3-Pyridyl, oder Thienyl, wie 2-Thienyl, und der andere unsubstituiertes oder durch Niederalkyl mit 1 bis 4 C-Atomen, wie Methyl, Niederalkoxy mit 1 bis 4 C-Atomen, wie Methoxy, und/oder Halogen mit Atomnummer bis und mit 35, wie Chlor oder vor allem Fluor, substituiertes Phenyl bedeutet, R₃ Wasserstoff oder in zweiter Linie Niederalkyl mit 1 bis 4 C-Atomen, wie Methyl, bedeutet, n für 0, 1 oder 2 steht, und R₄ Niederalkyl mit 1 bis 4 C-Atomen, wie Aethyl, darstellt, und ihre Salze, sowie diese Verbindungen enthaltende pharmazeutische Präparate und ihre Verwendung.

Die Erfindung betrifft insbesondere Verbindungen der Formel I, worin einer der Reste R₁ und R₂ unsubstituiertes Pyridyl, wie 3-Pyridyl, bedeutet und der andere unsubstituiertes oder in zweiter Linie durch Niederalkyl mit 1 bis 4 C-Atomen, z.B. Methyl, Niederalkoxy mit 1 bis 4 C-Atomen, z.B. Methoxy, oder Halogen, z.B. Chlor oder Brom, substituiertes Phenyl bedeutet, R₃ Wasserstoff oder Niederalkyl mit 1 bis 4 C-Atomen, z.B. Methyl oder Aethyl, darstellt, n für 0 steht, und R₄ Wasserstoff, Niederalkyl mit 1 bis 4 C-Atomen, z.B. Methyl oder Aethyl oder die Hydroxygruppe in höherer als der a-Stellung tragendes Hydroxyniederalkyl mit 2 bis 4 C-Atomen, z.B. 2-Hydroxyäthyl, bedeutet, und ihre Säureadditionssalze, sowie diese Verbindungen enthaltende pharmazeutische Präparate und ihre Verwendung.

Die Erfindung betrifft in allererster Linie Verbindungen der Formel I, worin einer der Reste A₁ und R₂ Pyridyl, wie 3-Pyridyl, und der andere Phenyl bedeutet, R₃ Wasserstoff ist, n für 0 oder 1 steht, und R Niederalkyl mit 1 bis 4 C-Atome, wie Aethyl, bedeutet, und ihre Säureadditionssalze, sowie diese Verbindungen enthaltende pharmazeutische Präparate und ihre Verwendung.

Die Erfindung betrifft namentlich die in den Beispielen Genannten Verbindungen der Formel und ihre Säureadditionssalze.

Die Erfindung betrifft weiterhin Verfahren zur Herstellung von Verbindungen der Formel 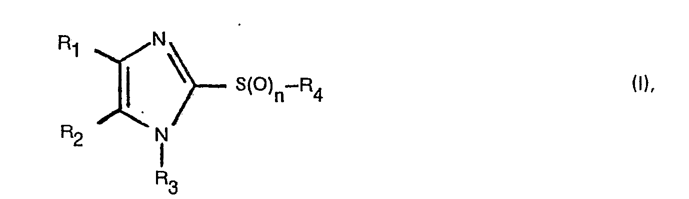 worin mindestens einer der Reste R₁ und R₂ eine unsubstituierte oder durch Niederalkyl, Niederalkoxy, Halogen, Amino, Niederalkylamino, Diniederalkylamino, Alkylen- bzw. 3-Aza-, 3-Oxa- oder 3-Thia- alkylenamino mit jeweils 5 oder 6 Ringgliedern und/oder Trifluormethyl C-substituierte und/oder durch Niederalkyl, Niederalkoxyniederalkyl, Hydroxyniederalkyl oder Oxy N-substituierte monocyclische, über ein C-Atom gebundene, ein Sauerstoff- oder Schwefelatom, ein Schwefel- und ein Stickstoffatom oder mindestens 2 Stickstoffatome aufweisende 5-gliedrige oder mindestens ein Stickstoffatom auf-' weisende 6-gliedrige Heteroarylgruppe und ein davon verschiedener Rest R₁ oder R₂ eine unsubstituierte oder durch Niederalkyl, Niederalkoxy, Halogen, Amino, Niederalkylamino, Diniederalkylamino, Alkylenamino bzw. 3-Aza-, 3-Oxa- oder 3-Thia-alkylenamino mit jeweils 5 oder 6 Ringgliedern, Nitro und/oder Trifluormethyl substituierte Phenylgruppe bedeutet, R₃ Wasserstoff oder Niederalkyl darstellt, n für 0, 1 oder 2 steht, und R₄ einen unsubstituierten oder durch unsubstituiertes oder durch Niederalkyl, Niederalkoxy, Halogen, Amino, Niederalkylamino, Diniederalkylamino, Alkylenamino bzw. 3-Aza-, 3-Oxa- oder 3-Thia-alkylenamino mit jeweils 5 oder 6 Ringgliedern, Nitro und/oder Trifluormethyl substituiertes Phenyl, substituierten Niederalkyl-, Niederalkenyl- oder Niederalkinylrest oder einen in höherer als der a-Stellung durch Hydroxy, Niederalkoxy, Niederalkylthio oder eine unsubstituierte oder durch Niederalkyl, Niederalkoxy, Halogen, Amino, Niederalkylamino, Diniederalkylamino, Alkylenamino bzw. 3-Aza-, 3-Oxa- oder 3-Thia-alkylenamino mit jeweils 5 oder 6 Ringgliedern, Nitro und/oder Trifluormethyl, substituierte Phenoxy- oder Phenylthiogruppe substituierten Niederalkylrest bedeutet, und ihrer Säureadditionssalze.

Die Verbindungen der Formel 1 und ihre Salze können nach an sich bekannten Methoden hergestellt werden, beispielsweise indem man
a) Verbindungen der Formeln 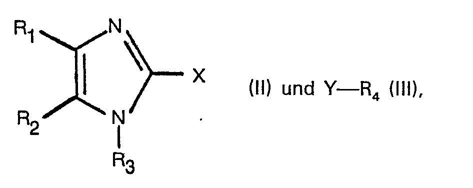 vorliegendes Mercapto und der andere einen gegen veräthertes Mercapto austauschbaren Rest bedeutet mit der Massgabe, dass in Verbindungen der Formel (II), worin X gegebenenfalls in Salzform vorliegendes Mercapto ist, Heteroarylreste R₁ und/oder R₂ von ein Schwefelatom aufweisenden 5- gliedrigen oder ein Stickstoffatom aufweisenden 6-gliedrigen Heteroarylrest oder Reste R₃ von Wasserstoff verschieden sind, wenn in Verbindungen der Formel (111) Y reaktionsfähiges verestertes Hydroxy und R₄ einen in 2-, 3- oder 4-Stellung durch Hydroxy einfach substituierten Niederalkylrest darstellt, oder
b) Verbindungen der Formeln II und III, worin einer der Reste X und Y eine Gruppe der Formel -S(O)ₙ‾Y₁, worin Y₁ eine reaktionsfähige veresterte Hydroxygruppe oder, wenn n für 0 steht, eine verätherte Mercaptogruppe oder, wenn n für 1 oder 2 steht, eine verätherte Hydroxygruppe ist, und der andere ein Metallradikal Y₂ darstellt, miteinander oder
c) eine Verbindung der Formel II, worin X die Mercaptogruppe darstellt, mit einem gegebenenfalls wie für R₄ angegeben substituierten Niederalken bzw.
d) eine Verbindung der Formel II, worin X eine in Salzform vorliegende Sulfin- oder Sulfonsäuregruppe bedeutet, mit einer Verbindung der Formel III, worin Y eine reaktionsfähige veresterte Hydroxygruppe bedeutet, umsetzt oder
e) eine Verbindung der Formel 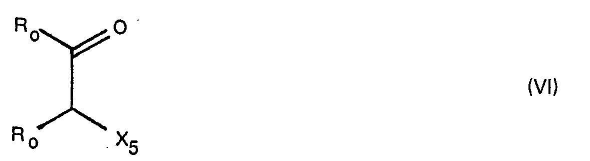 worin einer der Reste Rₐ eine Gruppe R₁ und der andere eine Gruppe R₂ darstellt und Xₛ eine reaktionsfähig veresterte Hydroxygruppe bedeutet, oder ein Säureadditionssalz davon, mit einem N‾R₃‾S‾R₄‾Isothioharnstoff umsetzt oder
f) eine Verbindung der Formel 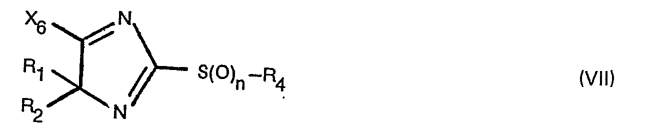 worin X₆ gegebenenfalls durch Phenyl, das seinerseits wie R₁ und R₂ substituiert sein kann, substituiertes Niederalkylthio ist, oder ein Säureadditionssalz davon, umlagert und gewünschtenfalls in einer gemäss der Verfahrensvarienten a) bis f) erhalten Verbindung der Formel 1, worin n für 0 steht, und R₁, R₂, R₃ und R₄ obige Bedeutungen haben, mit der Massgabe, dass in gemäss der Verfahrensvariante a) durch Umsetzung von Verbindungen der Formeln (II) und (III), worin X gegebenenfalls in Salzform vorliegendes Mercapto und Y reaktionsfähiges verestertes Hydroxy ist, erhaltenen Verbindungen der Formel (I) Heteroarylreste R₁ und/oder R₂ von ein Schwefel- oder Sauerstoffatom aufweisenden 5- gliedrigen oder ein Stickstoffatom aufweisenden 6-gliedrigen Heteroarylreste verschieden sind, wenn R₄ einen durch ein oder zwei Hydroxygruppen, oder ein Niederalkoxygruppe substituierten oder durch gegebenenfalls wie angegeben substituiertes Phenyl substituierten Niederalkyl- oder unsubstituierten Niederalkinylrest darstellt, die Thiogruppe zur Sulfinyl- oder Sulfonylgruppe oxidiert und/oder in gemäss einer der Verfahrensvariante a) bis f) erhaltenen Verbindungen, worin R₁ und/oder R₂ mindestens ein Stickstoffatom >N aufweist, dieses N-oxidiert oder in gemäss einer der Verfahrensvariante b) oder f) erhaltenen Verbindungen, worin n für 1 oder 2 steht, die Sulfinyl- oder Sulfonylgruppe zur Thiogruppe und/oder gemäss der Verfahrensvariante a) bis f) erhaltenen Verbindungen, worin R₁ und R₂ ein N-oxidiertes Stickstoffatom aufweist, dieses zu einer Gruppe >N reduziert, eine Verfahrensgemäss erhaltene Verbindung der Formel I, worin R₃ Wasserstoff darstellt, N-niederalkyliert, in verfahrensgemäss erhaltenen Verbindungen der Formel I Reste R₁, R₂ und/oder Phenyl-, Phenoxy- oder Phenylthiogruppen als Bestandteil von R₄ gegebenenfalls zusätzliche C-Substituenten einführt und/oder eine verfahrensgemäss erhaltene freie Verbindung in ein Säureadditionssalze oder ein verfahrensgemäss erhaltenes Säureadditionssalz in die freie Verbindung oder in ein anderes Salz überführt.

In Salzform vorliegende Mercapto- oder Sulfinsäuregruppen sind beispielsweise in Alkalimetall-oder Ammoniumsalzform, z.B. als Natrium-, Kalium- oder Ammoniumsalz, vorliegende Mercapto- oder Sulfinsäuregruppen.

Gegen veräthertes Mercapto austauschbare Reste X bzw. Y sind beispielsweise Halogenatome, z.B. Chlor, Brom oder Jod, gegen die Gruppe -SR₄ austauschbare Reste X sind ferner Sulfonylgruppen, vor allem von organischen Sulfonsäuren abgeleitete Sulfonylgruppen, z.B. Methan-, Aethan-, Benzol-, p-Brombenzol- oder p-Toluolsulfonyl. Gegen 1‾R₃‾4‾R₁‾5‾R₂‾Imidazolyl-(2)-thio austauschbare Reste Y sind ferner andere reaktionsfähig veresterte Hydroxygruppen als Halogen, wie mit Schwefelsäure oder mit einer organischen Sulfonsäure, z.B. mit Methan-, Aethan-, Benzol-, p-Brombenzol- oder p-Toluolsulfonsäure, veresterte Hydroxygruppen.

Reaktionsfähig veresterte Hydroxygruppen Y bzw. Y1 sind beispielsweise Halogenatome, wie Chlor, Brom oder Jod, oder Sulfonyloxygruppen, vorzugsweise Sulfonyloxygruppen der Formel ‾SO₂‾R₄ in Gruppen Y der Formel ‾S(O)n‾Y₁, worin n für 2 steht. Verätherte Mercaptogruppen Y₁ sind in Gruppen X der Formel -S(O)ₙ‾Y₁, worin n für 0 steht, beispielsweise 1-R₃-4-R₁-5-R₂-Imidazolyl-2-thioreste und in Gruppen Y der Formel S(O)ₙ‾Y₁, worin n für 0 steht, beispielsweise solche der Formel ‾SR₄. Veräthertes Hydroxy ist beispielsweise mit einem aromatischen Alkohol, wie einem gegebenenfalls substituierten Phenol veräthertes Hydroxy, in Gruppen der Formel ‾S(O)ₙ‾Y₁, worin n für 1 steht, ferner Niederalkoxy. Metallradikale Y₂ sind beispielsweise solche der Formeln―M¹, -M"/2 oder -M"-Hal, worin M' ein Alkalimetallatom, z.B. Lithium oder Natrium, und M" ein Erdalkalimetallatom, z.B. Magnesium, Cadmium oder Zink, bedeutet.

Die Umsetzung gemäss den Verfahrensvarianten a)-d) kann in üblicher, insbesondere in der aus der Literatur für analoge Umsetzungen bekannten Weise durchgeführt werden, erforderlichenfalls in Gegenwart eines katalytischen Mittels, bei der Umsetzung von Mercaptoverbindungen der Formel II mit Alkenen oder Phenylalkenen, beispielsweise saurer Mittel, wie Lewis-Säuren, z.B. von Eisen-II-Salzen oder Bortrifluorid, von Peroxiden, z.B. von Di-tert.-butylperoxid, oder unter Einwirkung von UV-Licht, z.B. der Wellenlänge von etwa 200 bis 350 um. Bei der Durchführung der genannten Reaktionen arbeitet man jeweils vorzugsweise in einem Lösungsmittel, ausgehend von Verbindungen der Formeln II und 111, worin einer der Reste X und Y ein Metallradikal und der andere eine Gruppe der Formel ‾S(O)ₙ‾Y₁ ist, sowie bei der Umsetzung von Mercaptoverbindungen der Formel II mit Alkenen oder Phenylalkenen der Formel III, beispielsweise in Aether, Tetrahydrofuran oder Dioxan oder bei der Umsetzung von Mercaptanen bzw. Mercaptiden oder Sulfonsäuresalzen der Formel II mit reaktionsfähigen Estern, z.B. Halogeniden, der Formel III beispielsweise in einem Alkohol, z.B. in Methanol, Aethanol, Aethylenglykol oder Aethylenglykolmonomethyläther, jeweils vorteilhaft unter einer Inertgasatmosphäre, z.B. unter Stickstoff, und erforderlichenfalls bei erhöhter Temperatur, z.B. in der Siedehitze.

Eine bevorzugte Ausführungsform der vorstehenden Verfahrensvariante a) bzw. d) besteht z.B. darin, dass man ein gegebenenfalls in einer der genannten Salzformen vorliegendes 2-Mercapto- oder 2-Sulfoimidazolderivat der Formel It in einem Niederalkanol, z.B. in Methanol oder Aethanol, mit einem Chlor-, Brom- oder Jodwasserstoff- oder Schwefelsäureester der Formel Il umsetzt.

In einer bevorzugten Ausführungsform der Verfahrensvariante b) setzt man beispielsweise eine Verbindung der Formel II, worin X ein Metallradikal Y₂, vorzugsweise Lithium, bedeutet, und R₃ vorzugsweise von Wasserstoff verschieden ist, z.B. in einem offenkettigen oder cyclischen aliphatischen Aether, z.B. in Diäthyläther, tert..₋Butylmethyläther, Tetrahydrofuran oder Dioxan, mit einer Verbindung der Formel III um, worin Y eine Gruppe der Formel -S-S-R₄, Halogen-S-R₄ oder ‾S(O)₂‾O-S(O)₂‾R₄ bedeutet.

Die Ausgangsstoffe sind teilweise bekannt. Neue Ausgangsstoffe können nach an sich bekannten Methoden hergestellt werden.

Die als Ausgangsstoffe genannten Verbindungen der Formel II, in denen X Mercapto bedeutet, d.h. 1-R₃-4-R₁-5-R₂-2-Mercapto-imidazole bzw. 1-R₃-4-R₁-5-R₂-imidazolin-thione, worin R₁, R₂ und R₃ die eingangs unter der Formel 1 angegebenen Bedeutungen haben, kann man beispielsweise herstellen, indem man eine Verbindung der Formel 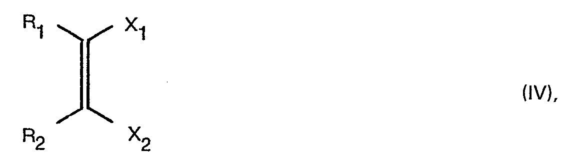 worin Xᵢ eine Gruppe der Formel -NH-C(=S)-NHR₃ und X₂ Hydroxy oder X₂ eine Gruppe der Formel ‾NR₃‾C(=S)‾NH₂ und X₁ Hydroxy bedeutet, oder ein gegebenenfalls in ketalisierter Form vorliegendes Tautomeres davon intramolekular cyclisiert.

Tautomere von Verbindungen der Formel IV sind vorzugsweise die zu den der Formel IV entsprechenden Enolen tautomeren Ketone. Diese können mit Niederalkanolen oder Niederalkandiolen, z.B. mit Methanol, Aethanol oder Aethylen-oder 1,3-Propylenglykol, ketalisiert sein.

Die intramolekulare Cyclisierung kann in üblicher, insbesondere in der in der Literatur für analoge Reaktionen beschriebenen, Weise erfolgen, beispielsweise in einem Lösungsmittel, wie Wasser oder einem Alkohol, z.B. in Wasser, Aethanol, Butanol, Aethylenglykol oder Aethylenglykolmonomethyläther, erforderlichenfalls in Gegenwart eines sauren Kondensationsmittels, wie einer Mineralsäure, z.B. von Chlorwasserstoffsäure, und/oder bei erhöhter Temperatur, z.B. bei Siedetemperatur.

Die Ausgangsstoffe der Formel IV ihrerseits können nach an sich bekannten Methoden erhalten werden, wobei man sie vorteilhaft in situ herstellt und ohne Isolierung cyclisiert. Dazu geht man vorzugsweise von einer Verbindung der Formel R₁‾CO‾CH(NHR₃)‾R₂ (IVa) oder einem Säureadditionssalz derselben aus und setzt diese mit Rhodanwasserstoffsäure oder einem Metallrohodanid um. Dabei wird intermediär eine Verbindung der Formel IV gebildet, die erfindungsgemäss cyclisiert. Vorteilhaft ist insbesondere die Umsetzung eines Hydrohalogenides, z.B. des Hydrochlorides, einer Verbindung der Formel IVa mit einem Alkalimetall- oder Ammoniumrhodanid, z.B. mit Natrium- oder Kaliumrhodanid, in wässriger Lösung, erforderlichenfalls unter Erwärmen auf 60 bis 100°C.

Eine Variante dieses Verfahrens besteht darin, dass man in analoger Weise eine Verbindung der Formel R₁‾CH(NH₂)‾CO‾R₂ (IVb) oder ein Säureadditionssalz davon mit einem Isothiocyanat der Formel R₃‾N=C=S (lVc) umsetzt.

Die Ausgangsstoffe der Formel IV kann man ferner herstellen indem man eine Verbindung der Formel R°-CO-CHOH-R° (IVd), oder eine durch a-Halogenierung einer Verbindung der Formel R_{°}-GO-CH₂ R_{°} (IVe), z.B. mit Brom in Essigsäure, erhältliche Verbindung der Formel R°‾CO‾CH(Halogen)‾R° (IVf), worin eine der Gruppen Rₒ den Rest R, und die andere den Rest R₂ darstellt, in üblicher Weise mit einer Thioharnstoffverbindung der Formel R₃NH-CS-NH_{Z} (IVg) oder einer diesen in situ erzeugenden Verbindung, z.B. einem R₃-Ammoniumrhodanid, umsetzt. Bei erhöhten Temperaturen, z.B. bei 100‾250°, cyclisiert die dabei primär gebildete Verbindung der Formel IV in erfindungsgemässer Weise. Dabei können, sofern R₁ und R₂ verschieden sind und R₃ nicht Wasserstoff darstellt, nach Massgabe der Reaktivitäten der individuellen Komponente der Formel IVc und/oder der Reaktionsbedingungen, beide oder nur eines der möglichen Isomeren, d.h. ein 1-R₃-4-R₁-5-R₂-2(3H)-imidazolin-2-thion und-oder ein 1-R₃-4-R₂-5-R₁-2(3H)-imidazolin-2-thion, erhalten werden, die gegebenenfalls in üblicher Weise, z.B. durch fraktionierte Kristallisation oder chromatographisch, getrennt werden können.

Die Ausgangsstoffe der Formel IVa ihrerseits können in an sich bekannter Weise hergestellt werden, beispielsweise indem man eine Verbindung der Formel R₁‾CO‾CH₂‾R₂ oximiert, das Oxim mit Toluolsulfochlorid in Pyridin in den Oximester überführt, diesen der Neber'schen Oxi-Aminketon-Umlagerung unterwirft und gewünschtenfalls in die erhaltene Verbindung der Formel R₁‾CO‾CH(NH₂)‾R₂ einen Rest R₃ einführt, z.B. durch Umsetzung mit einem Niederalkylbromid oder -jodid.

Ausgangsstoffe der Formel IVb kann man in analoger Weise herstellen, wobei man von einer Verbindung der Formel R₁‾CH₂‾CO‾R₂ ausgeht.

Ausgangsstoffe der Formel IVd, in denen R₁ und R2 gleich Reste Rₒ bedeuten, können ferner hergestellt werden durch, z.B. mit Kaliumcyanid in Aethanol/Wasser oder Tetrabutylammoniumcyanid in Wasser bewirkte, Selbstkondensation eines Aldehydes der Formel Ro‾CHO (lVh).

Die Verbindungen der Formel II, in denen X Mercapto ist, können ferner erhalten werden, indem man ein entsprechendes, in 2-Stellung unsubstituiertes Imidazolderivate der Formel 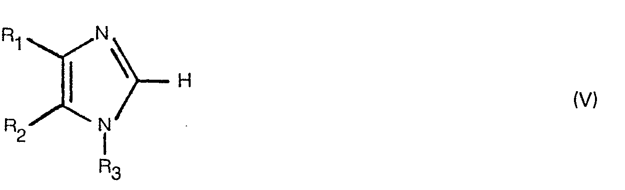 in Tetramethylensulfon mit Schwefel auf etwa 150-250°C, z.B. auf etwa 200°C, erhitzt.

Ausgangsstoffe der Formel II, worin X ein Metallradikal Y₂ bedeutet, werden vorzugsweise in situ hergestellt, beispielsweise durch Umsetzung einer Verbindung der Formel V mit einer metallorganischen Verbindung der Formel R-Y₂ (VI), worin R ein aliphatischer Rest, vorzugsweise Niederalkyl ist, beispielsweise mit Butyllithium oder Methyl- oder Butylmagnesiumbromid. Aus den so erhältlichen magnesiumorganischen Verbindungen können durch Umsetzung mit Zinkchlorid oder Cadmiumdichlorid sodann Verbindungen der Formel Il erhalten werden, worin Y₂ eine Gruppe Zn-Halogen bzw. Cd-Halogen ist.

Die Ausgangsstoffe der Formel V können ihrerseits hergestellt werden, indem man eine Verbindung der Formel Ro‾CO‾CH(Halogen)‾R° (IVf), worin einer der Reste Rₒ eine Gruppe R₁ und der andere eine Gruppe R₂ darstellt, mit Formamid erhitzt, vorzugsweise auf Siedetemperatur. Die Verbindungen der Formel IVf können ihrerseits erhalten werden, indem man eine Verbindung der Formel R₁‾CH=O bzw. R₂CH=O (Va) in Gegenwart eines Diniederalkylamin-, z.B. von Dimethylaminhydrochlorid, mit einem Alkalimetallcyanid umsetzt, das erhaltene 2-R,- bzw. 2-R₂₋Diniederalkylamino- acetonitril (Vb) in Dimethylformamid mit Natriumhydrid und anschliessend mit einer Verbindung der Formel R₂- bzw. R₁‾CH₂-Halogen (Vc) behandelt, das Kondensationsprodukt durch mehrstündiges Erhitzen in einem Gemisch aus konzentrierter Salzsäure und Chloroform hydrolysiert und die erhaltene Verbindung der Formel Ro‾CO‾CH₂‾R° (IVe), z.B. mittels Brom in Essigsäure oder Kupfer-II-bromid in Essigsäureäthylester halogeniert.

Ausgangsstoffe der Formel II, worin X eine gegebenenfalls veresterte oder anhydridisierte Sulfogruppe, wie Sulfo, Niederalkoxy- oder Phenoxysulfonyl oder eine Sulfonyloxysulfonylgruppe bedeutet, können beispielsweise erhalten werden, indem man in einer Verbindung der Formel II, worin X Mercapto ist, die Mercaptogruppe zur Sulfogruppe oxidiert und diese gewünschtenfalls, z.B. mit Phosphorpentachlorid, halogeniert und die Halogensulfonylgruppe mit einem Alkohol, z.B. Niederalkanol oder Phenol, in verestertes Sulfo überführt oder in üblicher Weise, z.B. durch Umsetzung mit einem Sulfonsäuresalz anhydridisiert. Analog kann man auch Sulfinsäureester der Formel Il herstellen, wobei man die Oxidation des Mercaptans auf der Sulfinsäurestufe anhält. Verbindungen der Formel II, worin X eine Gruppe -S-S-R₄ ist, können durch milde Oxidation von Mecaptanen der Formel II, z.B. mittels Eisen-III-chlorid oder Luftsauerstoff, erhalten werden.

Bei der Verfahrensvariante e) kann die Verbindung der Formel VI, worin R₁ und/oder R₂ eine basische Gruppe darstellt, auch in Form eines Säureadditionssalzes vorliegen.

Die Umsetzung gemäss der Verfahrensvariantec e) erfolgt in üblicher Weise, vorzugsweise indem man den N‾R₃‾S‾R₄‾Isothioharnstoff und gegebenenfalls die Verbindung der Formel VII, beispielsweise durch Einwirkung einer Base, wie eines Alkalimetallhydroxides oder -carbonates, z.B. von Natrium- oder Kaliumhydroxid oder -carbonat, oder einer Stickstoffbase, z.B von Ammoniak, eines Triniederalkylamins, wie Triäthylamin oder N,N-Diisopropyläthylamin oder Pyridin, in situ aus einem N‾R₃‾S‾R₄‾Isothiuroniumsalz, wie -alkosulfat oder -halogenid, z.B. -bromid oder -chlorid, und einer gegebenenfalls in Salzform vorliegenden Verbindung der Formel VI, freisetzt, wobei ausgehend von in Salzform vorliegenden Ausgangsstoffen der Formel VI 3, sonst 2 äquivalente Basen benötigt werden, vorteilhaft in einem Lösungsmittel, wie einem Niederalkanol, z.B. in Methanol, Aethanol, Butanol oder Amylalkohol, erforderlichenfalls bei erhöhter Temperatur, z.B. in der Siedehitze.

Die Ausgangsstoffe der Formel VI können nach an sich bekannten Methoden hergestellt werden, beispielsweise indem man eine Verbindung der Formel R₁‾CH=O bzw. R₂‾CH=O (Va) in Gegenwart eines Diniederalkylamin-, z.B. von Dimethylaminhydrochlorid, mit einem Alkalimetallcyanid umsetzt, das erhaltene 2-Rᵢ- bzw. 2-R₂₋Diniederalkylaminoacetonitril (Vb) in Dimethylformamid mit Natriumhydrid und anschliessend mit einer Verbindung der Formel R₂- bzw. R₁‾CH₂-Halogen (Vc) behandelt, das Kondensationsprodukt durch mehrstündiges Erhitzen in einem Gemisch aus konzentrierter Salzsäure und Chloroform hydrolysiert und die erhaltene Verbindung der Formel Ro‾CO‾CH₂‾R° (IVe), z.B. mittels Brom in Essigsäure oder Kupfer-II-bromid in Essigsäureäthylester, halogeniert.

Die Umlagerung gemäss der Verfahrensvariante f) kann in üblicher Weise erfolgen, erforderlichenfalls in Gegenwart eines Katalysators, wie einer Lewissäure, z.B. von Aluminiumtrichlorid, und vorteilhaft in einem Lösungsmittel, z.B. in Benzol, Toluol, N,N-Dimethylanilin, Anisol, Tetralin, Chlorbenzol, Pyridin oder Dekalin.

Die Ausgangsstoffe der Formel VII können in an sich bekannter Weise hergestellt werden, beispielsweise indem man eine Verbindung der Formel R₁‾CO‾CO‾R₂ (Vlla) zunächst mit Thioharnstoff umsetzt, das erhaltene S-R₁-5-R₂₋2-thiohydantoin mit Phosphorpentasulfid in das entsprechende 5-R₁-5-R₂-di-thiohydantoin überführt und dieses mit einem gegebenenfalls wie für X₆ angeben substituierten Niederalkylhalogenid weiterumsetzt.

In erhaltenen Verbindungen der Formel I kann man im Rahmen der gegebenen Definition Substituenten einführen, umwandeln oder abspalten.

So kann man beispielsweise anstelle eines Wasserstoffatoms R₃ durch Umsetzung mit einem den Rest R₃ einführenden Mittel einen organischen Rest R₃ einführten. Solche Mittel sind beispielsweise reaktionsfähige Ester, wie Halogenwasserstoffsäureester, z.B. Chlor-, Brom- oder Jodwasserstoffsäureester, organische Sulfonsäureester, z.B. Methan-, Aethan-, Benzol-, p-Bromobenzol- oder p-Toluolsulfonsäureester, oder Schwefelsäureester entsprechender Niederalkanole R₃0H. Die Umsetzung mit diesen Mitteln erfolgt in üblicher Weise, z.B. in Gegenwart eines basischen Kondensationsmittels, wie einem Alkalimetallhydroxid oder -carbonat, z.B. Natrium -oder Kaliumhydroxid oder Natrium- oder Kaliumcarbonat, einem Alkoholat, z.B. Alkalimetallniederalkanolat, wie Natriummethanolat, ferner von Natriumhydrid, vorteilhaft in einem inerten Lösungsmittel, z.B. in Dimethylformamid oder N-Methylpyrrolidon.

Ferner kann man in Verbindungen der Formel Reste ‾S(O)n‾R₄, worin n für 0 steht, zu den entsprechenden Sulfinyloder Sulfonylresten, worin n für 1 oder 2 steht, Reste ‾S(O)n‾R₄, worin n für 1 steht, zu den entsprechenden Sulfonylresten, worin n 2 bedeutet, und/oder Heteroarylreste R₁ und/oder R₂ mit mindestens einem freien Ringstickstoffatom >N, wie Pyridylreste, N-oxidieren. Die Oxidation erfolgt vorzugsweise durch Einwirkung eines geeigneten Oxidationsmittels, vorteilhaft in einem diesem gegenüber inerten Lösungsmittel, erforderlichenfalls unter Kühlen oder Erwärmen, z.B. im temperaturbereich von etwa -30°C bis +100°C, vorzugsweise bei etwa 0° bis 60°C, in einem geschlossenen Gefäss und/oder unter Inertgas, wie Stickstoff. Geeignete Oxidationsmittel sind beispielsweise Peroxyverbindungen, wie Wasserstoffperoxid, organische Hydroperoxide, z.B. tert.-Butylhydroperoxid, organische Persäuren, wie aromatische oder aliphatische Percarbonsäuren, z.B. m-Chlorperoxybenzoesäure, Peroxyessigsäure oder Permonophthalsäure, oxidierende Schwermetallverbindungen, wie Chrom-VI- oder Mangan-IV- bzw. Mangan-VII-verbindurigen, z.B. Chromtrioxid, Chromsäure, Mangandioxid oder Kaliumpermanganat, oxidierende anorganische Sauerstoffsäuren, wie Sauerstoffsäuren des Stickstoffs, der Halogene oder Chalkogene, oder deren Anhydride oder Salze, z.B. Salpetersäure, Distickstofftetroxid, Selendioxid oder Natriummetaperjodat, ferner Ozon. Geeignete Lösungsmittel sind beispielsweise halogenierte Kohlenwasserstoffe, wie Halogenalkane, z.B. Kohlenstofftetrachlorid, Chloroform oder Methylenchlorid, oder Carbonsäuren, wie Alkansäuren, z.B. Essigsäure, oder deren Anhydride.

In einer bevorzugten Ausführungsform dieses Oxidationsverfahrens kann man beispielsweise Thioäther der Formel I, worin n für 0 steht, und/oder einer der Reste R₁ und/oder R₂ ein unsubstituiertes Ringstickstoffatom >N aufweist, durch Umsetzung mit einer organischen Persäure, z.B. mit m-Chlorperbenzoesäure, in einem Halogenalkan, z.B. in Chloroform, zu den entsprechenden Sulfinyl- oder Sulfonyl-verbindungen, worin n für 1 oder 2 steht, und gegebenenfalls am Stickstoffatom >N oxidieren.

In einer anderen bevorzugten Ausführungsform kann man Thioäther der Formel I, worin n für 0 steht, durch Behandeln mit Natriummetaperjodat, vorzugsweise in einem Halogenalkan, z.B. in Kohlenstofftetrachlorid oder Chloroform, selektiv zu den entsprechenden Sulfonxiden, in denen n für 1 steht, oder diese mit Wasserstoffperoxid in Essigsäure zu Sulfonen, worin n für 2 steht, oxidieren.

Umgekehrt kann man in Verbindungen der Formel I, worin n für 1 oder 2 steht und/oder Heteroarylreste R₁ und/oder R₂ N-oxidiert sind, die Gruppe ‾S(O)‾R₄ bzw. -S(O)₂‾R₄ zu der entsprechenden Gruppe S(O)o‾R₄ und/oder N-oxidierten Ringstickstoff jN-O zu :>N reduzieren. Die Reduktion erfolgt durch Behandeln mit üblichen Reduktionsmitteln, z.B. mit nascierendem oder katalytisch aktiviertem Wasserstoff, wie Eisen oder Zink und Säure, wie Salzsäure, oder mit Wasserstoff in Gegenwart von Raney-Nickel, vorteilhaft in einem inerten Lösungsmittel, wie einem Niederalkanol oder mit Leichtmetallhydriden bzw. Dileichtmetallhydriden, z.B. mit Alkalimetallaluminium-oder Alkalimetallborhydriden, z.B. mit Natriumborhydrid oder Lithiumaluminiumhydrid, vorteilhaft in einem inerten Lösungsmittel, wie einem Aether, z.B. Diäthyläther oder Tetrahydrofuran, oder zur selektiven Reduktion von Gruppen >N-0 mit einer Phosphor-III-verbindung, wie einem Phosphin, z.B. Triphenylphosphin oder Tri-n-butylphosphin, oder einem Phosphorigsäureester, wie einem Triniederalkylphosphit, z.B. mit Trimethyl- oder Triäthylphosphit.

Ferner kann man in die Reste R₁ und R₂ sowie in Phenyl-, Phenoxy- und Phenylthiogruppen als Bestandteile von R₄ gegebenenfalls zusätzliche C-Substituenten einführen. So kann man in üblicher Weise halogenieren, z.B. durch Umsetzung mit Chlor oder Brom in Gegenwart von Eisen oder mittels N-Chlorsuccinimid. Ferner kann man in üblicher Weise, z.B. durch Umsetzung mit einem Niederalkylhalogenid, Niederalkanol oder Niederalken in Gegenwart von Aluminiumtrichlorid niederalkylieren. Weiterhin kann man in üblicher Weise, z.B. mittels Salpetersäure/Schwefelsäure, nitrieren.

Weiterhin kann man Nitrogruppen in üblicher Weise, wie mit nascierendem Wasserstoff, z.B. mit Eisen/Salzsäure, zu Amino reduzieren.

Aminogruppen können ferner in üblicher Weise durch Umsetzung mit einem Niederalkylierrungsmittel, wie einem der genannten in Gegenwart eines basischen Kondensationsmittels niederalkyliert werden.

Ferner können erhaltene freie Verbindungen in an sich bekannter Weise in Säureadditionssalze überführt werden z.B. durch Umsetzen einer Lösung der freien Verbindung in einem geeigneten Lösungsmittel oder Lösungsmittelgemisch mit einer der vorgenannten Säuren oder mit einer Lösung davon, oder mit einem geeigneten Anionenaus-tauscher.

Erhaltene Säureadditionssalze können in an sich bekannter Weise in die freien Verbindungen umgewandelt werden, z.B. durch Behandeln mit einer Base, wie einem Alkalimetallhydroxid, einem Metallcarbonat oder -hydrogencarbonat, oder Ammoniak, oder mit einem geeigneten Anionenaustauscher.

Erhaltene Säureadditionssalze können in an sich bekannter Weise in andere Säureadditionssalze überführt werden, z.B. durch Behandlung mit einem Anionaustauscher oder durch Behandlung eines Salzes einer anorganischen Säure mit einem geeigneten Metallsalz, wie einem Natrium-, Barium- oder Silberhalz, einer Säure in einem geeigneten Lösungsmittel, in welchem ein sich bildendes anorganisches Salz unlöslich ist und damit aus dem Reaktionsgemisch ausscheidet.

Die Verbindungen, einschliesslich ihrer Salze können auch in der Form der Hydrate erhalten werden oder das zur Kristallisation verwendete Lösungsmittel einschliessen.

Infolge der engen Beziehung zwischen den neuen Verbindungen in freier Form und in Form ihrer Salz sind im vorausgegangenen und nachfolgend unter den freien Verbindungen oder ihren Salzen sinn-und zweckgemäss gegebenenfalls auch die entsprechenden Salze bzw. freien Verbindungen zu verstehen.

Die Erfindung betrifft auf diejenigen Ausführungsformen des Verfahrens, bei denen man von einer auf beliebiger Verfahrensstufe als Zwischenprodukt erhältlichen Verbindung ausgeht und die fehlenden Verfahrensschritte ausführt, oder wobei ein Ausgangsstoff unter den Reaktionsbedingungen gebildet oder in Form eines Derivats davon, gegebenenfalls eines Salzes, verwendet wird.

Beim Verfahren der vorliegenden Erfindung werden vorzugsweise solche Ausgangsstoffe verwendet, welche zu den eingangs als besonders wertvoll geschilderten Verbindungen führen.

Bei den erfindungsgemässen pharmazeutischen Präparaten, welche Verbindungen der Formel I bzw. 11 oder pharmazeutisch verwendbare Salze davon enthalten, handelt es sich um solche zur ent= eralen wie oralen oder rektalen, und parenteralen Verabreichung sowie zur topischen Anwendung an Warmblüter(n), welche den pharmakologischen Wirkstoff allein oder zusammen mit einem pharmazeutisch anwendbaren Trägermaterial enthalten. Die Dosierung des Wirkstoffs hängt von der Warmblüter-Spezies, dem Alter und dem individuellen Zustand, sowie von der Applikationsweise ab. Im Normalfall ist für einen etwa 75 kg schweren Warmblüter bei oraler Applikation eine ungefähre Tagesdosis von etwa 30-300 mg, vorteilhaft in mehreren gleichen Teildosen verteilt, zu veranschlagen.

Die neuen pharmazeutischen Präparate enthalten z.B. von etwa 10% bis etwa 80%, vorzugsweise von etwa 20% bis etwa 60% des Wirkstoffs. Erfindungsgemäss pharmazeutische Präparate zur enteralen bzw. parenteralen Verabreichung sind z.B. solche in Dosiseinheitsformen wie Dragees, Tabletten, Kapseln oder Suppositorien, ferner Ampullen. Diese werden in an sich bekannter Weise, z.B. mittels konventioneller Misch-, Granulier-, Gradier-, Lösungs- oder Lyophilisierungsverfahren hergestellt. So kann man pharmazeutische Präparate zur oralen Anwendung erhalten, indem man den Wirkstoff mit festen Trägerstoffen kombiniert, ein erhaltenes Gemisch gegebenenfalls granuliert, und das Gemisch bzw. Granulat, wenn erwünscht oder notwendig, nach Zugabe von geeigneten Hilfsstoffen, zu Tabletten oder Dragee-Kernen verableitet.

Geeignete Trägerstoffe sind insbesondere Füllstoffe, wie Zucker, z.B. Lactose, Saccharose, Mannit oder Sorbit, Cellulosepräparate und/oder Calciumphosphate, z.B. Tricalciumphosphat oder Calciumhydrogenphosphat, ferner Bindemittel, wie Stärkekleister unter Verwendung z.B. von Mais- Weizen-, Reis- oder Kartoffelstärke, Gelatine, Traganth, Methylcellulose und/oder Polyvinylpyrrolidon, und/oder, wenn erwünscht, Sprengmittel, wie die obengenannten Stärken, ferner Carboxymethylstärke, quervernetztes Polyvinylpyrrolidon, Agar, Alginsä₋ure oder ein Salz davon, wie Natriumalginat. Hilfsmittel sind in erster Linie Fliessregulier- und Schmiermittel, z.B. Kieselsäure, Talk, Stearinsäure oder Salze davon, wie Magnesium- oder Calciumstearat, und/oder Polyäthylenglykol. Dragee-Kerne werden mit geeigneten, gegebenenfalls Magensaft-resistenten Ueberzügen versehen, wobei man u.a. konzentrierte Zuckertösungen, welche gegebenenfalls arabischen Gummi, Talk, Polyvinylpyrrolidon, Polyäthylenglycol und/oder Titandioxid enthalten, Lacklösungen in geeigneten organischen Lösungsmitteln oder Lösungsmittelgemischen oder, zur Herstellung von Magensaft-resistenten Ueberzügen, Lösungen von geeigneten Cellulosepräparaten, wie Acetylcellulosephthalat oder Hydroxypropylmethylcellulosephthalat, verwendet. Den Tabletten oder Dragee-Ueberzügen können Farbstoffe oder Pigmente, z.B. zur Identifizierung oder zur Kennzeichnung verschiedener Wirkstoffdosen, beigefügt werden.

Weitere oral anwendbare pharmazeutische Präparate sind steckkapseln aus Gelatine, sowie weiche, geschlossene Kapseln aus Gelatine und einem Weichmacher, wie Glycerin oder Sorbitol. Die Steckkapseln können den Wirkstoff in Form eines Granulats, z.B. im Gemisch mit Füllstoffen, wie Lactose, Bindemitteln, wie Stärken, und/oder Geitmitteln, wie Talk oder Magnesiumstearat, und gₑ- gebenenfalls von Stabilisatoren, enthalten. In weichen Kapseln ist der Wirkstoff vorzugsweise in geeigneten Flüssigkeiten wie fetten Oelen. Paraffinöl oder flüssigen Polyäthylenglycolen, gelöst oder suspendiert, wobei ebenfalls Stabilisatoren zugefügt sein können.

Als rektal anwendbare pharmazeutische Präparate kommen z.B. Suppositorien in Betracht, welche wie fette Oele, z.B. Sesamöl, oder synthetische Fettsäureester, z.B. Aethyloleat oder Triglyceride, grundmasse eignen sich z.B. natürliche oder synthetische Triglyceride, Paraffinkohlenwasserstoffe, Polyäthylenglycole oder höhere Alkanole. Ferner können auch Gelatine-Rektalkapseln verwendet werden, die eine Kombination des Wirkstoffs mit einer Grundmasse enthalten; als Grundmassenstoffe kommen z.B. flüssige Triglyceride, Polyäthylenglykole oder Paraffinkohlenwasserstoffe in Frage.

Zur parenteralen Verabreichung eigenen sich in erster Linie wässrige Lösungen eines Wirkstoffs in wasserlöslicher Form, z.B. eines wasserlöslichen Salzes, ferner Suspensionen des Wirkstoffs, wie entsprechende ölige Injektionssuspensionen, wobei man geeignete lipophile Lösungsmittel oder Vehikel, wie fette Oele, z.B. Sesamöl, oder synthetische Fettsäureester, z.B. Aethyloeat oder Triglyceride, verwendet, oder wässrige Injektionssuspensionen, welche viskositätserhöhende Stoffe, z.B. NatriumCarboxymethylcellulose, Sorbit und/oder Dextran und gegebenenfalls auch Stabilisatoren enthalten.

Als topisch ansendbare pharmazeutische Präparate kommen in erster Linie Creme, Salben, Pasten, Schäume, Tinkturen und Lösungen, in Frage, die von etwa 0,5 bis etwa 20% des Wirkstoffs enthalten.

Creme sind Oel-in-Wasser-Emulsionen, die mehr als 50% wasser aufweisen. Als ölige Grundlage verwendet man in erster Linie Fettalkohole, z.B. Lauryl-, Cetyl- oder Stearylalkohol, Fettsäuren, z.B. Palmitin- oder Stearinsäure, flüssige bis feste Wachse, z.B. Isopropylmyristat, Wollwachs oder Bienenwachs, und/oder Kohlenwasserstoffe, z.B. Vaseline (Petrolatum) oder Parffinöl. Als Emulgatoren kommen oberflächenaktive Substanzen mit vorwiegend hydrophilen Eigenschaften in Frage, wie entsprechende nichtionische Emulgatoren, z.B. Fettsäureester von Polyalkoholen oder Aethylenoxidaddukte davon, wie Polyglycerinfettsäureester oder Polyoxyäthylensorbitan-fettsäureester (Tweens), ferner Polyoxyäthylenfettalkoholäther oder -fettsäureester, oder entsprechende ionische Emulgatoren, wie Alkalimetallsalze von Fettalkoholsulfaten, z.B. Natriumlaurylsulfat, Natriumcetylsulfat oder Natriumstearylsulfat, die man üblicherweise in Gegenwart von Fettalkoholen, z.B. Cetylalkohol oder Stearylalkohol, verwendet. Zusätze zur Wasserphase sind u.a. Mittel, welche die Austrocknung der Creme vermindern, z.B. Polyalkohole, wie Glycerin, Sorbit, Propylenglykol und/oder Polyäthylenglykole, ferner Konservierungsmittel, Riechstoffe, etc.

Salben sind Wasser-in Oel-Emulsionen, die bis zu 70%, vorzugsweise jedoch von etwa 20% bis etwa 50% Wasser oder wässrige Phasen enthalten. Als Fettphase kommen in erster Linie Kohlenwasserstoffe, z.B. Vaseline, Paraffinöl und/oder Hartparaffine in Frage, die zur Verbesserung des Wasserbindungsvermögens vorzugsweise geeignete Hydroxyverbindungen, wie Fettalkohole oder Ester davon, z.B. Cetylalkohol oder Wollwachsalkohole, bzw. Wollwachs enthalten. Emulgatoren sind entsprechende lipophile Substanzen, wie Sorbitan-fettsäureester (Spans), z.B. Sorbitanoleat und/oder Sorbitanisostearat. Zusätze zur Wasserphase sind u.a. Feuchthaltungsmittel, wie Polyalkohole, z.B. Glycerin, Propylengylkol, Sorbit und/oder Poläthylenglykol, sowie Konservierungsmittel, Riechstoffe, etc.

Fettsalben sind wasserfrei und enthalten als Grundlage insbesondere Kohlenwasserstoffe, z.B.. Paraffin, Vaseline und/oder flussige Paraffine, ferner natürliche oder partialsynthetische Fett, z.B. Kokosfettsäuretriglycerid, oder vorzugsweise gehärtete Oele, z.B. hydriertes Erdnuss- oder Rizinusöl, ferner Fettsäurepartialester des Glycerins, z.B. Glycerinmono- und -distearat, sowie z.B. die im Zusammenhang mit den Salben erwähnten, die Wasseraufnahmefähigkeit steigernden Fettalkohole, Emulgatoren und/oder Zusätze.

Pasten sind Creme und Salben mit sekretabsorbierenden Puderbestandteilen, wie Metalloxiden, z.B. Titanoxid oder Zinkoxid, ferner Talk und/oder Aluminiumsilikate, welche die Aufgabe haben, vorhandene Feuchtigkeit oder Sekrete zu binden.

Schäume werden aus Druckbehältern verabreicht und sind in Aerosolform vorleigende flüssige Oel-in-Wasser-Emulsionen, wobei halogenierte Kohlenwasserstoffe, wie Chlorfluorniederalkane, z.B. Dichlordifluormethan und Dichlortetrafluoräthan, als Treibmittel verwendet werden. Als Oelphase verwendet man u.a. Kohlenwasserstoffe, z.B. Paraffinöl, Fettalkohole, z.B. Cetylalkohol, Fettsäureester, z.B. Isopropylmyristat, und/oder andere Wachse. Als Emulgatoren verwendet man u.a. Gemische von solchen mit vorwiegend hydrophilen Eigenschaften, wie Polyoxyäthylen-sorbitan-fettsäureester (Tweens), und solchen mit vorwiegend lipophilen Eigenschaften, wie Sorbitanfettsäureester (Spans). Dazu kommen die üblichen Zusätze, wie Konservierungsmittel, etc.

Tinkturen und Lösungen weisen meistens eine wässerig-äthanolische Grundlage auf, der u.a. Polyalkohole, z.B. Glycerin, Gykole, und/oder Polyäthylenglykol, als Feuchthaltemittel zur Herabsetzung der Verdunstung, und rückfettende Substanzen, wie Fettsäureester mit niedrigen Polyäthylenglycolen, d.h. im wässrigen Gemisch lösliche, lipophile Substanzen als Ersatz für die der Haut mit dem Aethanol entzogenen Fettsubstanzen, und, falls notwendig, andere Hilfs- und Zusatzmittel beigegeben sind.

Die Herstellung der topisch verwendbaren pharmazeutischen Präparate erfolgt in an sich bekannter Weise, z.B. durch Lösen oder Suspendieren des Wirkstoffs in der Grundlage oder in einem Teil davon, falls notwendig. Bei Verarbeitung des Wirkstoffs als Lösung wird dieser in der Regel von der Emulgierung in einer der beiden Phasen gelöst; bei Verarbeitung als Suspension wird er nach der Emulgierung mit einem Teil der Grundlage vermischt und dann dem Rest der Formulierung beigegeben.

Die vorliegende Erfindung betrifft ebenfalls die Verwendung der Verbindungen der Formeln I und II und der Salze von solchen Verbindungen mit salzbildenden Eigenschaften, vorzugsweise zur Behandlung von Entzündungen, in erster Linie von entzündlichen chronischen Erkrankungen des rheumatischen Formenkreises, besonders der chronischen Arthritis.

Die nachfolgenden Beispiele illustrieren die oben beschriebene Erfindung; sie sollen jedoch diese in ihrem Umfang in keiner Weise einschränken. Temperaturen sidn in Celsiusgraden angegeben.

### Beispiel 1

0,8 g Natrium werden in 200 ml Aethanol gelöst. Die Lösung wird mit 8 g 2-Mercapto-4(5)-phenyl-5(4)-(3-pyridyl)-imidazol versetzt und zum Rückfluss erhitzt. Nachdem eine klare Lösung entstanden ist, werden 2,3 ml 2-Chloräthanol zugetropft und das Reaktionsgemisch 16 Stunden zum Rückfluss erhitzt. Das Lösungsmittel wird am Vakuum abgedampft und der Rückstand zwischen Wasser und Essigester verteilt. Die organischen Phasen werden mit Wasser und gesättigter Kochsalzlösung gewaschen, über Natriumsulfat getrocknet und eingedampft. Als Rückstand erhält man kristallines 2-(2-Hydroxyäthylthio)-5(4)-phenyl-4(5)-(3-pyridyl)-imidazol, das nach Umkristallisieren aus Essigester/Petroläther bei 128-130°C schmilzt.

Das Ausgangsmaterial kann beispielsweise folgendermassen hergestellt werden.

10,8 g Benzyl-(3-pyridyl)-keton werden zusammen mit 40 ml Pyridin und einer Lösung von 8 g Hydroxylaminhydrochlorid in 15 ml Pyridin 6 Stunden bei 100° gerührt. Das Reaktionsgemisch wird auf Eis/Wasser gegossen und 15 Minuten nachgerührt. Die ausgefallenen Kristalle werden abgenutscht, mit Wasser gewaschen und im Hochvakuum getrocknet. Man erhält das Benzyl-(3-pyridyl)-keon-oxim vom Smp. 122-126°.

Zu einer bei -10° gerührten Lösung von 8,5 g Benzyl-(3-pyridyl)-keton-oxim in 20 ml Pyridin wird innerhalb von 5 Minuten eine Lösung von 7,7 g p-Toluolsulfochlorid in 15 ml Pyridin zugetropft. Das Reationsgemisch wird 24 Stunden im Eisschrank aufbewahrt und denn auf Eis/Wasser gegossen. Nach einigem Rühren und Verreiben erstarrt das ausgefallene Oel zu Kristallen. Diese werden abgenutscht, mit Wasser gewaschen und im Hockvakuum getrocknet. Man erhält den Benzyl-(3-pyridyl)-keton-oxim-p-toluolsulfonsäureester, der ohne weitere Reinigung in der nächsten Stufe eingesetzt wird.

11,6 g roher Benzyl-(3-pyridyl)-keton-oxim-p-toluolsulfoester werden in 90 ml absolutem Aethanol suspendiert. Dann werden bei 0° unter Rühren die Lösung von 3,7 g Kalium-tert.-butylat in 30 ml absolutem Aethanol zugetropft. Das Reaktionsgemisch wird 2 Stunden bei 0° gerührt. Die Suspension wird abgenutscht und das Filtrat, welches das gewünschte a-Amino-benzyl-(3-pyridyl)-keton enthält, sofort in der nächsten Stufe weiterumgesetzt.

3,6 g Natriumthiocyanat werden in 60 ml Aethanol gelöst und mit 4,5 ml konzentrierter Salzsäure versetzt. Die Suspension wird abgenutscht und das Filtrat wird zusammen mit der alkoholischen Lösung von a-Amino-benzyl-(3-pyridyl)-keton 18 Stunden zum Rückfluss erhitzt. Nach dem Abkühlen lässt sich aus dem Reaktionsgemisch das rohe 2-Mercapto-4(5)-phenyl-5(4)-(3-pyridyl)-imidazol abnutschen. Das Filtrat enthält weiteres Produkt. Das Rohprodukt wird aus Dimethylformamid-Wasser umkristallisiert und schmilzt dann bei 290-300°.

### Beispiel 2

5 g 2-Mercapto-4(5)-phenyl-5(4)-(3-pyridyl)-imidazol werden in 50 ml Methanol und 10 ml 2n-Natronlauge gelöst. Man tropft 2,85 g Methyljodid hinzu, lässt 2 Stunden bei Raumtemperatur rühren, fügt 50 ml Wasser hinzu, saugt ab und wäscht den Rückstand mit Wasser nach. Man erhält rohes 2-Methylthio-4(5)-phenyl-5(4)-(3-pyridyl)-imidazol, welches, aus einem Gemisch von Isopropanol und Petroläther (8:5 Vol.-Teile) umkristallisiert, bei 193-194° schmilzt.

### Beispiel 3

8 g 2-Mercapto-4(5)-phenyl-5(4)-(3-pyridyl)-imidazol werden in 40 ml Methanol und 16 ml 2n-Natronlauge gelöst. Zu der Lösung werden 4,55 g Methyljodid zugetropft. Man lässt 2 Stunden bei Raumtemperatur nachrühren, fügt 40 ml Wasser hinzu, saugt ab und wäscht mit Wasser nach. Man erhält ein Gemisch von 1-Methyl-2-methylthio-4-phenyl-5-(3-pyridyl)-imidazol und 1-Methyl-2-methylthio-5-phenyl-4-(3-pyridyl)-imidazol. Dieses kann chromatographisch in die Komponente aufgetrennt werden. So wird an einer Silicagelsäule mit Chloroform zunächst die eine Komponente vom Smp. 141-143° und dann mit Chloroform/Essigester (8:2 Vol.-Teile) die andere Komponente vom Smp. 125-127° eluiert.

### Beispiel 4

In analoger Weise wie in den Beispielen 1-3 beschrieben kann man ferner herstellen:
2-(2-Hydroxyäthylthio)-1-methyl-4-phenyl-5-(3-pyridyl)-imidazol,
2-(2-Hydroxyäthylthio)-1 -methyl-5-phenyl-4-(3-pyridyl)-imidazol,
2-(2-Methoxyäthylthio)-4(5)-phenyl-5(4)-(3-pyridyl)-imidazol,
2-(2-Methoxyäthylthio)-1 -methyl-4-phenyl-5-(3-pyridyl)-imidazol,
2-(2-Methoxyäthylthio)-1-methyl-5-phenyl-4-(3-pyridyl)-imidazol,
2-(2-Methylthioäthylthio)-4(5)-phenyl-5(4)-(3-pyridyl)-imidazol,
1-Methyl-2-(2-methylthioäthylthio)-4-phenyl-5-(3-pyridyl)-imidazol,
1-Methyl-2-(2-methylthioäthylthio)-5-phenyl-4-(3-pyridyl)-imidazol,
2-Aethylthio-4(5)-phenyl-5(4)-(3-pyridyl)-imidazol,
2-Propylthio-4(5)-phenyl-5(4)-(3-pyridyl)-imidazol,
2-lsopropyl-4(5)-phenyl-5(4)-(3-pyridyl)-imidazol,
2-Methylthio-4,5-bis-(2-thienyl)-imidazol und
2-Aethylthio-4,5-bis-(2-thienyl)-imidazol.

### Beispiel 5

In analoger Weise wie in Beispiel 2 beschrieben können ausgehend von jeweils 7,6 g 2-Mercapto-4(5)-phenyl-5(4)-(3-pyridyl)-imidazol das 2-Aethylthio-4(5)-phenyl-5(4)-(3-pyridyl)-imidazol, Smp. 196-198° (aus Isopropanol/Petroläther), 2-Propylthio-4(5)-phenyl-5(4)-(3-pyridyl)-imidazol, Smp. 143-144° (aus Isopropanol/Petroläther) und 2-lsopropylthio-4(5)-phenyl-5(4)-(3-pyridyl)-imidazol, Smp. 182-184° (aus Isopropanol/Petroläther), hergestellt werden.

### Beispiel 6

Eine Suspension von 8 g 2-Methylthio-4(5)-phenyl-5(4)-(3-pyridyl)-imidazol in 400 ml Chloroform wird unter Rühren tropfenweise mit einer Lösung von 6,3 g 85%-iger m-Chlorperbenzoesäure in 70 ml Chloroform versetzt. Die entstandene Lösung wird über Nacht stehengelassen und dann nacheinander mit Natriumhydrogencarbonatlösung und Wasser gewaschen, über Natriumsulfat getrocknet und zur Trockne eingedampft. Der Rückstand wird zweimal aus Isopropanol/Petroläther umkristallisiert. Man erhält das 2-Methansulfinyl-4(5)-phenyl-5(4)-(3-pyridyl)-imidazol vom Smp. 166-169°.

In analoger Weise erhält man ausgehend von 2-Aethylthio-4(5)-phenyl-5(4)-(3-pyridyl)-imidazol das 2-Aethansulfinyl-4(5)-phenyl-5(4)-(3-pyridyl)-imidazol vom Smp. 162-164°.

### Beispiel 7

1,0 g 2-Aethansulfinyl-4(5)-phenyl-5(4)-(3-pyridyl)-imidazol werden mit 8 ml Essigsäure und 0,38 ml 30%-igem Wasserstoffperoxid versetzt und über Nacht bei 70° gerührt. Man lässt abkühlen, neutralisiert mit Natronlauge und saugt ab. Man erhält das 2-Aethansulfonyl-4(5)-phenyl-5(4)-[3-(1-Oxidopyridinio)]-imidazol, vom Smp. 208-210°.

### Beispiel 8

2,86 g a-Brombenzyl-(3-pyridyl)-ketonhydrobromid und 1 g S-Methylisothiuroniumbromid werden in 30 ml Acetonitril suspendiert, mit 3,1 g N,N-Diisopropyl-äthylamin versetzt und über Nacht gerührt. Die rotorange Suspension wird filtriert und das Filtrat unter vermindertem Druck eingedampft. Der Rückstand wird in Chloroform aufgenommen, über Natriumsulfat getrocknet, eingedampft und aus Chloroform/Petroläther umkristallisiert. Man erhält das 2-Methylthio-4(5)-phenyl-5(4)-(3-pyridyl)-imidazol vom Smp. 193-194°.

Das Ausgangsmaterial kann z.B. folgendermassen hergestellt werden:
20 g Benzyl-(3-pyridyl)-keton in 200 ml Essigsäure werden tropfenweise mit einer Lösung von 17 g Brom in 150 ml Essigsäure versetzt und über Nacht rühren gelassen. Das ausgefallene a-Brombenzyl-(3-pyridyl)-keton-hydrobromid wird abfiltriert und kann ohne weitere Reinigung verwendet werden.

### Beispiel 9

In analoger Weise wie in den Beispielen 1 bis 8 beschrieben kann man ferner
2-Aethylthio-4(5)-(p-fluorphenyl)-5(4)-(2-thienyl)-imidazol,
2-Methylthio-4(5)-(p-fluorphenyl)-5(4)-(2-thienyl)-imidazol und
2-Propylthio-4(5)-(p-fluorphenyl)-5(4)-(2-thienyl)-imidazol herstellen.

### Beispiel 10

Tabletten, enthaltend 25 mg Wirkstoff, z.B. 2-Methylthio-4(5)-phenyl-5(4)-(3-pyridyl)-imidazol, können folgendermassen hergestellt werden:

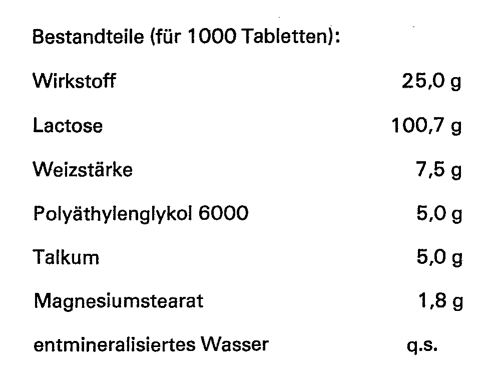

Herstellung

Sämtliche festen Ingredienzien werden zunächst durch ein Sieb mit 0,6 mm Maschenweite getrieben. Dann wird der Wirkstoff, die Lactose, das Talkum, das Magnesiumstearat und die Hälfte der Stärke vermischt. Die andere Hälfte der Stärke wird in 40 ml Wasser suspendiert und diese Suspension zu einer siedenden Lösung des Polyäthylenglykols in 100 ml Wasser hinzugegeben. Der erhaltene Stärkekleister wird zu der Hauptmenge hinzugegeben und das Gemisch, wenn nötig unter Hinzufügen von Wasser, granuliert. Das Granulat wird über Nacht bei 35° getrocknet, durch ein Sieb mit 1,2 mm Maschenweite getrieben und zu beidseitig konkaven Tabletten von etwa 6 mm Durchmesser verpresst.

In analoger Weise können auch Tabletten, enthaltend jeweils 25 mg einer anderen der in den Beispielen 1 bis 4 genannten Verbindungen der Formel I hergestellt werden.

### Beispiel 11

Kautabletten, enthaltend 30 mg Wirkstoff, z.B. 2-Methylthio-4(5)-phenyl-5(4)-(3-pyridyl)-imidazolin, können z.B. folgendermassen hergestellt werden:

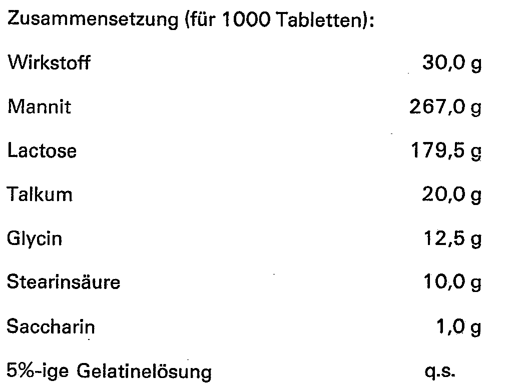

Herstellung

Alle feste Ingredienzien werden zunächst durch ein Sieb mit 0,2.5 mm Maschenweite getrieben. Der Mannit und die Lactose werden gemischt, unter Hinzufügen von Gelatinelösung granuliert, durch ein Sieb mit 2 mm Maschenweite getrieben, bei 50° getrocknet und nochmals durch ein Sieb mit 1,7 mm Maschenweite getrieben. Der Wirkstoff, das Glycin und das Saccharin werden sorgfältig vermischt, der Mannit, das Lactosegranulat, die Stearinsäure und das Talkum hinzugegeben, das Ganze gründlich vermischt und zu beidseitig konkaven Talbetten von etwa 10 mm Durchmesser mit Bruchrille auf der Oberseite verpresst.

In analoger Weise können auch Kautabletten, enthaltend jeweils 30 mg einer anderen der in den Beispielen 1 bis 4 genannten Verbindungen der Formel I hergestellt werden.

### Beispiel 12

Tabletten enthaltend 100 mg Wirkstoff, z.B. 2-Methylthio-4(5)-phenyl-5(4)-(3-pyridyl)-imidazol, können folgendermassen hergestellt werden:

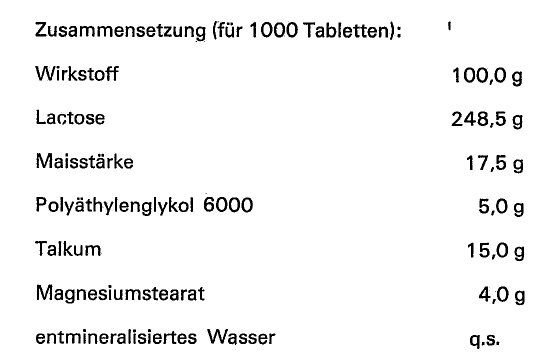

Herstellung

Die festen Ingredienzien werden zunächst durch ein Sieb mit 0,6 mm Maschenweite getrieben. Dann werden Wirkstoff, Lactose, Talkum Magnesiumstearat und die Hälfte der Stärke innig vermischt. Die andere Hälfte der Stärke wird in 65 ml Wasser suspendiert und diese Suspension zu einer siedenden Lösung des Polyäthylenglykols in 260 ml Wasser hinzugegeben. Der erhaltene Kleister wird zu den pulverförmigen Substanzen hinzugefügt, das Ganze vermischt und granuliert, erforderlichenfalls unter Zugabe von Wasser. Das Granulat wird über Nacht bei 35° getrocknet, durch ein Sieb mit 1,2 mm Maschenweite getrieben und zu beidseitig konkaven Tabletten von etwa 10 mm Durchmesser mit Bruchkerbe auf der Oberseite verpresst.

In analoger Weise können auch Tabletten, enthaltend 100 mg einer anderen Verbindung der Formel 1 gemäss dem Beispiel 1 hergestellt werden.

### Beispiel 13

In analoger Weise wie in den Beispielen 10 bis 12 beschrieben kann man ferner Tabletten mit jeweils 25 oder 100 mg bzw. Kautabletten mit 30 mg einer Verbindung gemäss einem der Beispiele 5 bis 8 herstellen.

### Beispiel 14

In analoger Weise wie in den Beispielen 1 bis 8 beschrieben kann man ferner
2-Aethansulfinyl-1 (5)-(p-fluorphenyl)-5(4)-(2-thienyl)-imidazol,
2-Aethansulfonyl-4(5)-(p-fluorphenyl)-5(4)-(2-thienyl)-imidazol und
2-Aethansulfonyl-4(5)-phenyl-5(4)-(3-pyridyl)-imidazol herstellen.

## Patentansprüche

1. Verbindungen der Formel 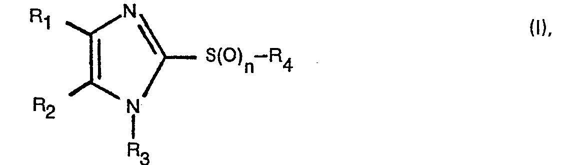 worin mindestens einer der Reste R₁ und R₂ eine unsubstituierte oder durch Niederalkyl, Niederalkoxy, Halogen, Amino, Niederalkylamino, Diniederalkylamino, Alkylen- bzw. 3-Aza-, 3-Oxa- oder 3-Thia- alkylenamino mit jeweils 5 oder 6 Ringgliedern und/oder Trifluormethyl C-substituierte und/oder durch Niederalkyl, Niederalkoxyniederalkyl, Hydroxyniederalkyl oder Oxy N-substituierte monocyclische, über ein C-Atom gebundene, ein Sauerstoff- oder Schwefelatom, ein Schwefel- und ein Stickstoffatom oder mindestens 2 Stickstoffatome aufweisende 5-gliedrige oder mindestens ein Stickstoffatom aufweisende 6-gliedrige Heteroarylgruppe. und ein davon verschiedener Rest R₁ oder R₂ eine unsubstituierte oder durch Niederalkyl, Niederalkoxy, Halogen, Amino, Niederalkylamino, Diniederalkylamino, Alkylenamino bzw. 3-Aza-, 3-Oxa- oder 3-Thia-alkylenamino mit jeweils 5 oder 6 Ringgliedern, Nitro und/oder Trifluormethyl substituierte Phenylgruppe bedeutet, R₃ Wasserstoff oder Niederalkyl darstellt, n für 0, 1 oder 2 steht und R₄ einen unsubstituierten oder durch unsubstituiertes oder durch Niederalkyl, Niederalkoxy, Halogen, Amino, Niederalkylamino, Diniederalkylamino, Alkylenamino, bzw. 3-Aza-, 3-Oxa- oder 3-Thia-alkylenamino mit jeweils 5 oder 6 Ringgliedern, Nitro und/oder Trifluormethyl substituiertes Phenyl substituierten Niederalkyl-, Niederalkenyl- oder Niederalkinylrest oder einen in höherer Stellung als der a-Stellung durch Hydroxy, Niederalkoxy, Niederalkylthio; eine unsubstituierte oder durch Niederalkyl, Niederalkoxy, Halogen, Amino, Niederalkylamino, Diniederalkylamino, Alkylenamino bzw. 3-Aza-, 3-Oxa- oder 3-Thia-alkylenamino mit jeweils 5 oder 6 Ringgliedern; Nitro·und/oder Trifluormethyl substituierte Phenoxy- oder Phenylthiogruppe substituierten Niederalkylrest bedeutet, mit der Massgabe, dass Heteroarylreste R₁ und/oder R₂ von ein Schwefelatom aufweisenden 5-gliedrigen und ein Stickstoffatom aufweisenden 6-gliedrigen Heteroarylresten oder Reste R₃ von Wasserstoff verschieden sind, wenn R₄ in 2-, 3- oder 4-Stellung durch eine Hydroxygruppe substituiertes Niederalkyl bedeutet, und mit der weiteren Massgabe, dass in Verbindungen der Formel I, worin n für 1 oder 2 steht, Heteroarylreste R₁ und/oder R₂ von ein Schwefel- oder Sauerstoffatom aufweisenden 5-gliedrigen und ein Stickstoffatom aufweisenden 6-gliedrigen Heteroarylresten verschieden sind, wenn R₄ durch ein oder zwei Hydroxygruppen, eine Niederalkoxygruppe oder durch gegebenenfalls wie angegeben substituiertes Phenyl substituiertes Niederalkyl oder unsubstituiertes Niederalkinyl darstellt, wobei "niedere" Reste bis und mit 7 C-Atome aufweisen, und ihre Säureadditionssalze.

2. Verbindungen gemäß Anspruch 1 worin mindestens einer der Reste R₁ und R₂ eine unsubstituierte oder durch Niederalkyl, Niederalkoxy, Halogen, Amino, Niederalkylamino, Diniederalkylamino, Alkylen- bzw. 5-Aza-, 3-Oxa- oder 3-Thia-alkylenamino mit jeweils 5 oder 6 Ringgliedern und/oder Trifluormethyl C-substituierte und/oder durch Niederalkyl, Niederalkoxyniederalkyl oder Hydroxyniederalkyl N-substituierte monocyclische, über ein C-Atom gebundene, ein Sauerstoff- oder Schwefelatom, ein Schwefel- und ein Stickstoffatom oder mindestens 2 Stickstoffatome aufweisende 5- gliedrige oder mindestens ein Stickstoffatom aufweisende 6-gliedrige Heteroarylgruppe und ein davon verschiedener Rest R₁ oder R₂ eine unsubstituierte oder durch Niederalkyl, Niederalkoxy, Halogen, Amino, Niederalkylamino, Diniederalkylamino, Alkylenamino bzw. 3-Aza-, 3-Oxa- oder 3-Thia- alkylenamino mit jeweils 5 oder 6 Ringgliedern, Nitro und/oder Trifluormethyl substituierte Phenylgruppe bedeutet, R₃ Wasserstoff oder Niederalkyl darstellt, n für 0 steht und R₄ einen unsubstituierten oder durch unsubstituiertes oder durch Niederalkyl, Niederalkoxy, Halogen, Amino, Niederalkylamino, Diniederalkylamino, Alkylenamino, bzw. 3-Aza₋, 3-Oxa- oder 3-Thia-alkylenamino mit jeweils 5 oder 6 Ringgliedern, Nitro und/oder Trifluormethyl substituiertes Phenyl substituierten Niederalkyl-, Niederalkenyl- oder Niederalkinylrest oder einen in höherer Stellung als der a-Stellung durch Hydroxy, Niederalkoxy, Niederalkylthio, eine unsubstituierte oder durch Niederalkyl, Niederalkoxy, Halogen, Amino, Niederalkylamino, Diniederalkylamino, Alkylenamino bzw. 3-Aza-, 3-Oxa- oder 3-Thia-alkylenamino mit jeweils 5 oder 6 Ringgliedern, Nitro und/oder Trifluormethyl substituierte Phenoxy- oder Phenylthiogruppe substituierten Niederalkylrest bedeutet, mit der Massgabe, dass Heteroarylreste R₁ und/oder R₂ von ein Schwefelatom aufweisenden 5-gliedrigen und ein Stickstoffatom aufweisenden 6-gliedrigen Heteroarylresten verschieden sind, wenn R₄ in 2-, 3- oder 4-Stellung durch eine Hydroxygruppe substituiertes Niederalkyl bedeuten, und ihre Säureadditionssalze.

3. Verbindungen gemäss Anspruch 1, worin mindestens einer der Reste R, und R₂ Furyl, Thienyl, Thiazolyl, Imidazolyl, Triazolyl, Tetrazolyl, Pyridyl, Pyrimidinyl oder Triazinyl bedeutet, wobei Ringkohlenstoffatome der genannten Reste R₁ und R₂ durch Niederalkyl, Niederalkoxy, Halogen mit und bis Atomnummer 35, Trifluormethyl, Amino, Niederalkylamino und/oder Alkylen- bzw. 3-Aza-, 3-Oxa- oder 3-Thia-alkylenamino mit jeweils 5 oder 6 Ringgliedern, Ringstickstoffatome von Pyrimidinyl-, Triazinyl-oder Pyridylresten ferner durch Oxy substituiert sein können, und ein davon verschiedener Rest R₁ oder R₂ Phenyl darstellt, welches durch Niederalkyl, Niederalkoxy, Halogen mit und bis Atomnummer 35, Trifluormethyl, Nitro, Amino, Niederalkylamino und/oder Alkylen- bzw. 3-Aza-, 3-Oxa- oder 3-Thia- alkylenamino mit jeweils 5 oder 6 Ringgliedern substituiert sein kann, R3 Wasserstoff oder Niederalkyl bedeutet, n für 0, 1 oder 2 steht, und R₄ einen unsubstituierten oder durch gegebenenfalls durch Niederalkyl, Niederalkoxy, Halogen mit und bis Atomnummer 35, Trifluormethyl, Nitro, Amino, Niederalkylamino und/oder Alkylen- bzw. 3-Aza-, 3-Oxa- oder 3-Thia-alkylenamino mit jeweils 5 oder 6 Ringgliedern substituiertes Phenyl substituierten Niederalkyl-, Niederalkenyl- oder Niederalkinylrest oder einen in höherer als der a-Stellung durch Hydroxy, Niederalkoxy, Niederalkylthio oder eine gegebenenfalls durch Niederalkyl, Niederalkoxy, Halogen mit und bis Atomnummer 35, Trifluormethyl, Nitro, Amino, Niederalkylamino und/oder Alkylen- ,bzw. 3-Aza-, 3-Oxa- oder 3-Thia-alkylenamino mit jeweils 5 oder 6 Ringgleidern substituierte Phenoxy- oder Phenylthiogruppe substituierten Niederalkylrest bedeutet, und ihre Säureadditionssalze.

4. Verbindungen gemäss Anspruch 2, worin mindestens einer der Reste R₁ und R₂ Furyl, Thienyl, Thiazolyl, Imidazolyl, Triazolyl, Tetrazolyl, Pyridyl, Pyrimidinyl oder Triazinyl bedeutet, wobei Ringkohlenstoffatome der genannten Reste R₁ und R₂, durch Niederalkyl, Niederalkoxy, Halogen mit und bis Atomnummer 35, Trifluormethyl, Amino, Niederalkylamino und/oder Alkylen- bzw. 3-Aza-, 3-Oxa- oder 3-Thia-alkylenamino mit jeweils 5 oder 6 Ringgliedern substituiert sein können, und ein davon verschiedener Rest R₂ oder R₃ Phenyl darstellt, welches durch Niederalkyl, Niederalkoxy, Halogen mit und bis Atomnummer 35, Trifluormethyl, Nitro, Amino, Niederalkylamino, und/oder Alkylen- bzw. 3-Aza-, 3-Oxa- oder 3-Thia-alkylenamino mit jeweils 5 oder 6 Ringgliedern substituiert sein kann, R₃ Wasserstoff oder Niederalkyl bedeutet, n für 0 steht, und R₄ einen substituierten oder durch gegenbenenfalls durch Niederalkyl, Niederalkoxy, Halogen mit und bis Atomnummer 35, Trifluormethyl, Nitro, Amino, Niederalkylamino und/oder Alkylen- bzw. 3-Aza-, 3-Oxa- oder 3-Thia-alkylenamino mit jeweils 5 oder 6 Ringgliedern substituiertes Phenyl substituierten Niederalkyl-, Niederalkenyl- oder Niederalkinylrest oder in höherer als der a-Stellung durch Hydroxy, Niederalkoxy, Niederaikylthio oder eine gegebenenfalls durch Niederalkyl, Niederalkoxy, Halogen mit und bis Atomnummer 35, Trifluormethyl, Nitro, Amino, Niederalkylamino und/oder Alkylen- bzw. 3-Aza-, 3-Oxa- oder 3-Thia- alkylenamino mit jeweils 5 oder 6 Ringgliedern substituierte Phenoxy- oder Phenylthiogruppe substituierten Niederalkylrest bedeutet, und ihre Säureadditionssalze.

5. Verbindungen gemäss Anspruch 2, worin einer der Reste R, und R₂ Pyridyl und der andere Phenyl bedeutet, wobei diese Reste unabhängig voneinander durch Niederalkyl mit 1 bis 4 C-Atomen, Niederalkoxy mit 1 bis 4 C-Atomen, Halogen bis und mit Atomnummer 35 und/oder N,N-Diniederalkylamino mit jeweils 1 bis 4 C-Atomen substituiert sein können, R₃ Wasserstoff oder Niederalkyl mit 1 bis 4 C-Atomen bedeutet, n für 0 steht, und R₄ Niederalkyl mit 1 bis 4 C-Atomen, Niederalkenyl mit 2 bis 4 C-Atomen, unsubstituiertes oder durch Niederalkyl mit 1 bis 4 C-Atomen, Niederalkoxy mit 1 bis 4 C-Atomen und/oder Halogen mit Atomnummer bis und mit 35 substituiertes Phenylniederalkyl mit 1 bis 4 C-Atomen im Alkylteil, die Hydroxygruppe(n) in höherer als der a-Stellung tragendes Mono- der Dihydroxyniederalkyl mit 2 bis 4 C-Atomen, die Alkoxy- oder Alkylthiogruppe in einer höheren als der a-Stellung tragendes Niederalkoxy- oder Niederalkylthioniederalkyl mit 1 bis 4 C-Atomen im Alkoxy bzw. Alkylthioteil und 2 bis 4 C-Atomen im Alkylteil bedeutet, und ihre Säureadditionssalze.

6. Verbindungen gemäss Anspruch 1, worin einer der Reste R, und R₂ unsubstituiertes Pyridyl oder Thienyl und der andere unsubstituiertes oder durch Niederalkyi mit 1 bis 4 C-Atomen, Niederalkoxy mit 1 bis 4 C-Atomen und/oder Halogen mit Atomnummer bis und mit 35 substituiertes Phenyl bedeutet, R₃ Wasserstoff oder Niederalkyl mit 1 bis 4 C-Atomen bedeutet, n für 0, 1 oder 2 steht, und R₄ Niederalkyl mit 1 bis 4 C-Atomen bedeutet, und ihre Säureadditionssalze.

7. Verbindungen gemäss Anspruch 2, worin einer der Reste R, und R₂ unsubstituiertes Pyridyl und der andere unsubstituiertes oder durch Niederalkyl mit 1 bis 4 C-Atomen, Niederalkoxy mit 1 bis 4 C-Atomen oder Halogen, substituiertes Phenyl bedeutet, R₃ Wasserstoff oder Niederalkyl mit 1 bis 4 C-Atomen darstellt, n für 0 steht, und R₄ Niederalkyl mit 1 bis 4 C-Atomen oder die Hydroxygruppe in höherer als der a-Stellung tragendes Hydroxyniederalkyl mit 2 bis 4 C-Atomen bedeutet, und ihre Säureadditionssalze.

8. Verbindungen gemäss Anspruch 1, worin einer der Reste R₁ und R₂ Pyridyl und der andere Phenyl bedeutet, R₃ Wasserstoff ist, n für 0 oder 1 steht, und R₄ Niederalkyl mit 1 bis 4 C-Atomen bedeutet, und ihre Säureadditionssalze.

9. Verbindungen gemäss einem der Ansprüche 1 bis 8 als antiinflammatorica und/oder antinociceptiva.

10. 2-Aethylthio-4(5)-phenyl-5(4)-(3-pyridyl)-imidazol oder ein Säureadditionssalz davon.

11. 2-Aethansulfinyl-4(5)-phenyl-5(4)-(3-pyridyl)-imidazol oder ein Säureadditionssalz davon.

12. 2-Aethylthio-4(5)-(p-fluorphenyl)-5(4)-(2-thienyl)-imidazol.

13. 2-(2-Hydroxyäthylthio)-4(5)-phenyl-5(4)-(3-pyridyl)-imidazol oder ein Säureadditionssalz davon.

14. 2-Methylthio-4(5)-phenyl-5(4)-(3-pyridyl)-imidazol oder ein Säureadditionssalz davon.

15. 2-(2-Methylthioäthylthio)-4(5)-phenyl-5(4)-(3-pyridyl)-imidazol oder ein Säureadditionssalz davon.

16. 2-(2-Methoxyäthylthio)-4(5)-phenyl-5(4)-(3-pyridyl)-imidazol oder ein Säureadditionssalz davon.

17. 2-Propylthio-4(5)-phenyl-5(4)-(3-pyridyl)-imidazol oder ein Säureadditionssalz davon.

18. 2-lsopropylthio-4(5)-phenyl-5(4)-(3-pyridyl)-imidazol oder ein Säureadditionssalz davon.

19. 2-Aethansulfonyl-4(5)-phenyl-5(4)-(3-pyridyl)-imidazol oder ein Säureadditionssalz davon.

20. 2-Aethansulfinyl-4(5)-(p-fluorphenyl)-(2-thienyl)-imidazol oder ein Säureadditionssalz davon.

21. 2-Aethansulfonyl-4(5)-(p-fluorphenyl)-(2-thienyl)-imidazol oder ein Säureadditionssalz davon.

22. 2-Aethylthio-4,5-bis-(2-thienyl)-imidazol oder ein Säureadditionssalz davon.

23. Verbindungen der Formel 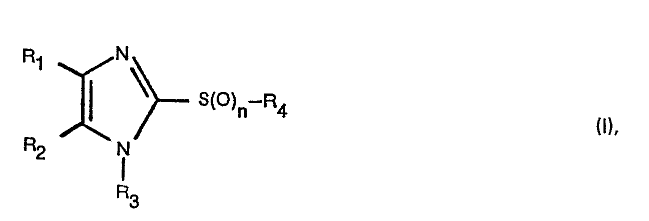 worin mindestens einer der Reste R₁ und R₂ eine unsubstituierte oder durch Niederalkyl, Niederalkoxy, Halogen, Amino, Niederalkylamino, Diniederalkylamino, Alkylen- bzw. 3-Aza-, 3-Oxa- oder 3-Thia- alkylenamino mit jeweils 5 oder 6 Ringgliedern und/oder Trifluormethyl C-substituierte und/oder durch Niederalkyl, Niederalkoxyniederalkyl, Hydroxyniederalkyl oder Oxy N-substituierte monocyclische, über ein C-Atom gebundene, ein Sauerstoff- oder Schwefelatom, ein Schwefei- und ein Stickstoffatom oder mindestens 2 Stickstoffatome aufweisende 5-gliedrige oder mindestens ein Stickstoffatom aufweisende 6-gliedrige Heteroarylgruppe und ein davon verschiedener Rest R₁ oder R₂ eine unsubstituierte oder durch Niederalkyl, Niederalkoxy, Halogen, Amino, Niederalkylamino, Diniederalkylamino, Alkylenamino bzw. 3-Aza-, 3-Oxa- oder 3-Thia-alkylenamino mit jeweils 5 oder 6 Ringgliedern, Nitro und/oder Trifluormethyl substituierte Phenylgruppe bedeutet, R₃ Wasserstoff oder Niederalkyl darstellt, n für 0, 1 oder 2 steht, und R₄ einen unsubstituierten oder durch unsubstituiertes oder durch Niederalkyl, Niederalkoxy, Halogen, Amino, Niederalkylamino, Diniederalkylamino, Alkylenamino bzw. 3-Aza-, 3-Oxa- oder 3-Thia-alkylenamino mit jeweils 5 oder 6-Ringgliedern, Nitro und/oder Trifluormethyl substituiertes Phenyl substituierten Niederalkyl-, Niederalkenyl- oder Niederalkinylrest oder einen in höherer als der a-Stellung durch Hydroxy, Niederalkoxy, Niederalkylthio, oder eine unsubstituierte oder durch Niederalkyl, Niederalkoxy, Halogen, Amino, Niederalkylamino, Diniederalkylamino, Alkylenamino bzw. 3-Aza-, 3-Oxa- oder 3-Thia-alkylenamino mit jeweils 5 oder 6 Ringgliedern, Nitro und/oder Trifluormethyl substituierte Phenoxy- oder Phenylthiogruppe substituierten Niederalkylrest bedeutet, mit der Massgabe, dass in Verbindungen der Formel I, worin n für 1 oder 2 steht, Heteroarylreste R₁ und/oder R₂ von ein Schwefel- oder Sauerstoffatom aufweisenden 5-gliedrigen und ein Stickstoffatom aufweisenden 6-qliedrigen Heteroarylresten verschieden sind, wenn R₄ einen durch ein oder zwei Hydroxygruppen, eine Niederalkoxygruppe oder durch gegebenenfalls wie angegeben substituiertes Phenyl substituierten Niederalkyl- oder unsubstituierten Niederalkinylrest darstellt, wobei "niedere" Reste bis und mit 7 C-atome aufweisen, und ihre pharmazeutisch verwendbaren Säureadditionssalze zur Behandlung entzündlicher Erkrankungen.

24. Verbindungen der Formel 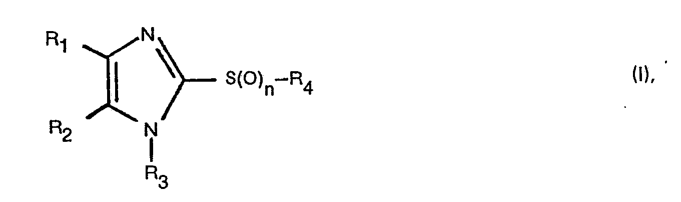 worin mindestens einer der Reste R₁ und R₂ eine unsubstituierte oder durch Niederalkyl, Niederalkoxy, Halogen, Amino, Niederalkylamino, Diniederalkylamino, Alkylen- bzw. 5-Aza-, 3-Oxa- oder 3-Thia- alkylenamino mit jeweils 5 oder 6 Ringgliedern und/oder Trifluormethyl C-substituierte und/oder durch Niederalkyl, Niederalkoxyniederalkyl oder Hydroxyniederalkyl N-substituierte monocyclische, über ein C-Atom gebundene, ein Sauerstoff- oder Schwefelatom, ein Schwefel- und Stickstoffatom oder mindestens 2 Stickstoffatome aufweisende 5-gliedrige oder mindestens ein Stickstoffatom aufweisende 6-gliedrige Heteroarylgruppe und ein davon verschiedener Rest R₁ oder R₂ eine unsubstituierte oder durch Niederalkyl, Niederalkoxy, Halogen, Amino, Niederalkylamino, Diniederalkylamino, Alkylenamino bzw. 3-Aza-, 3-Oxa- oder 3-Thia-alkylenamino mit jeweils 5 oder 6 Ringgliedern, Nitro und/oder Trifluormethyl substituierte Phenylgruppe bedeutet, R₃ Wasserstoff oder Niederalkyl darstellt, n für 0 steht und R₄ einen unsubstituierten oder durch unsubstituiertes oder durch Niederalkyl, Niederalkoxy, Halogen, Amino, Niederalkylamino, Diniederalkylamino, Alkylenamino, bzw. 3-Aza-, 3-Oxa- oder 3-Thia-alkylenamino mit jeweils 5 oder 6 Ringgliedern, Nitro und/oder Trifluormethyl substituiertes Phenyl substituierten Niederalkyl-, Niederalkenyl- oder Niederalkinylrest oder einen in höherer Stellung als der a-Stellung durch Hydroxy, Niederalkoxy, Niederalkylthio, eine unsubstituierte oder durch Niederalkyl, Niederalkoxy, Halogen, Amino, Niederalkylamino, Diniederalkylamino, Alkylenamino bzw. 3-Aza-, 3-Oxa- oder 3-Thia-alkylenamino mit jeweils 5 oder 6 Ringgliedern, Nitro und/oder Trifluormethyl substituierte Phenoxy- oder Phenylthiogruppe substituierten Niederalkylrest bedeutet, wobei "niedere" Rest bis und mid 7 C-Atome aufweisen, und ihre pharmazeutisch verwendbaren Säureadditionssalze zur Behandlung entzündticher Erkrankungen.

25. Verbindungen gemäss einem der Ansprüche 2, 4, 5, 7, 10, 13-18 und 22 zur Behandlung entzündlicher Erkrankungen.

26. Verbindungen gemäss einem der Ansprüche 1, 3, 6, 8, 9 und 19-21 zur Behandlung entzündlicher Erkrankungen.

27. Pharmazeutische Präparate enthaltend eine Verbindung gemäss einem der Ansprüche 2, 4, 5, 7, 10, 13-18, 22, 24 und 25 zusammen mit üblichen pharmazeutischen Hilfsstoffen.

28. Pharmazeutische Präparate enthaltend eine Verbindung gemäss einem der Ansprüche 1, 3, 6, 8, 9, 19-21, 23 und 26 zusammen mit üblichen pharmazeutischen Hilfsstoffen. -

29. Verfahren zur Herstellung von Verbindungen der Formel 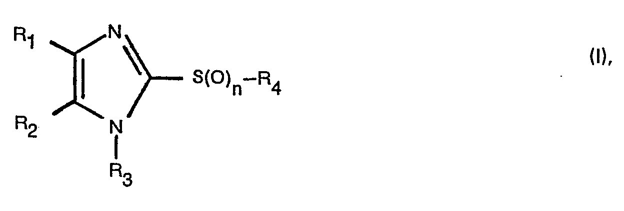 worin mindestens einer der Reste R₁ und R₂ eine unsubstituierte oder durch Niederalkyl, Niederalkoxy, Halogen, Amino, Niederalkylamino, Diniederalkylamino, Alkylen- bzw. 3-Aza-, 3-Oxa- oder 3-Thia- alkylenamino mit jeweils 5 oder 6 Ringgliedern und/oder Trifluormethyl C-substituierte und/oder durch Niederalkyl, Niederalkoxyniederalkyl, Hydroxyniederalkyl oder Oxy N-substituierte monocyclische, über ein C-Atom gebundene, ein Sauerstoff- oder Schwefelatom, ein Schwefel- und ein Stickstoffatom oder mindestens 2 Stickstoffatome aufweisende 5-gliedrige oder mindestens ein Stickstoffatom aufweisende 6-gliedrige Heteroarylgruppe und ein davon verschiedener Rest R₁ oder R₂ eine unsubstituierte oder durch Niederalkyl, Niederalkoxy, Halogen, Amino, Niederalkylamino, Diniederalkylamino, Alkylenamino bzw. 3-Aza-, 3-Oxa- oder 3-Thia-alkylenamino mit jeweils 5 oder 6 Ringgliedern, Nitro und/oder Trifluormethyl substituierte Phenylgruppe bedeutet, R₃ Wasserstoff oder Niederalkyl darstellt, n für 0, 1 oder 2 steht, und R₄ einen unsubstituieren oder durch unsubstituiertes oder durch Niederalkyl, Niederalkoxy, Halogen, Amino, Niederalkylamino, Diniederalkylamino, Alkylenamino bzw. 3-Aza-, 3-Oxa- oder 3-Thia-alkylenamino mit jeweils 5 oder 6 Ringgliedern, Nitro und/oder Trifluormethyl substituiertes Phenyl substituierten Niederalkyl-, Niederalkenyl- oder Niederalkinylrest oder einen in höherer als der a-Stellung durch Hydroxy, Niederalkoxy, Niederalkylthio oder eine unsubstituierte oder durch Niederalkyl, Niederalkoxy, Halogen, Amino, Niederalkylamino, Diniederalkylamino, Alkylenamino bzw. 3-Aza-, 3-Oxa- oder 3-Thia-alkylenamino mit jeweils 5 oder 6 Ringgliedern, Nitro und/oder Trifluormethyl, substituierte Phenoxy- oder Phertylthiogruppe substituierten Niederalkylrest bedeutet, und ihrer Säureadditionssalze, wobei "niedere" Reste bis und mit 7 C-Atome aufweisen, dadurch gekennzeichnet, dass man
a) Verbindungen der Formel 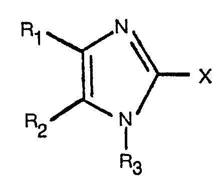 (II) und Y‾R₄ (III), worin einer der Reste X und Y gegebenenfalls in Salzform vorliegendes Mercapto und der andere einen gegen veräthertes Mercapto austauschbaren Rest bedeutet mit der Massgabe, dass in Verbindungen der Formel (II), worin X gegebenenfalls in Salzform vorliegendes Mercapto ist, Heteroarylreste R, und/ oder R₂ von ein Schwefelatom aufweisenden 5-gliedrigen und ein Stickstoffatom aufweisenden 6- gliedrigen Heteroarylrest oder Reste R₃ von Wasserstoff verschieden sind, wenn in Verbindungen der Formel (III) Y reaktionsfähiges verestertes Hydroxy und R₄ in 2-, 3- oder 4-Stellung durch Hydroxy einfach substituiertes Niederalkyl darstellt, oder
b) Verbindungen der Formeln 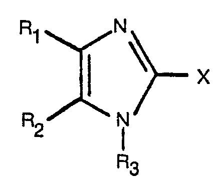 (II) und Y‾R₄ (111), worin einer der Reste X und Y eine Gruppe der Formel ‾S(O)ₙ‾Y₁, worin Y₁ eine reaktionsfähige veresterte Hydroxygruppe oder, wenn n für 0 steht, eine verätherte Mercaptogruppe oder, wenn n für 1 oder 2 steht, eine verätherte Hydroxygruppe ist, und der andere ein Metallradikal Y₂ darstellt, miteinander oder
c) eine Verbindung der Formel 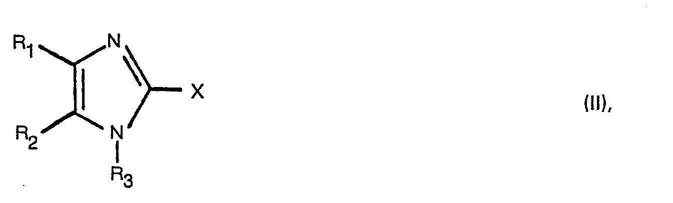 worin X die Mercaptogruppe darstellt, mit einem gegebenenfalls wie für Niederalkyl R₄ angegeben substituierten Niederalken umsetzt, bzw.
d) eine Verbindung der Formel 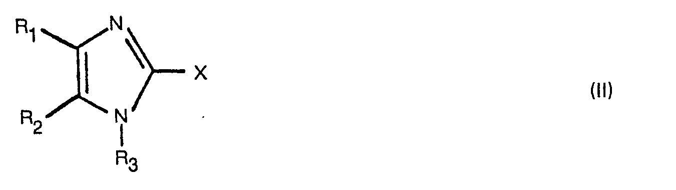 worin X eine in Salzform vorliegende Sulfin- oder Sulfonsäuregruppe bedeutet, mit einer Verbindung der Formel 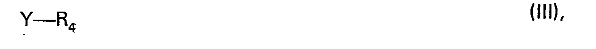 worin Y eine reaktionsfähige veresterte Hydroxygruppe bedeutet, umsetzt, oder
e) eine Verbindung der Formel 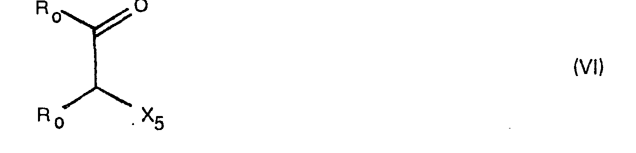 worin einer der Reste Ro eine Gruppe R₁ und der andere eine Gruppe R₂ darstellt und X₅ eine reaktionsfähig veresterte Hydroxygruppe bedeutet, oder ein Säureadditionssalz davon, mit einem N‾R₃‾S‾R₄-Isothioharnstoff umsetzt oder
f) eine Verbindung der Formel 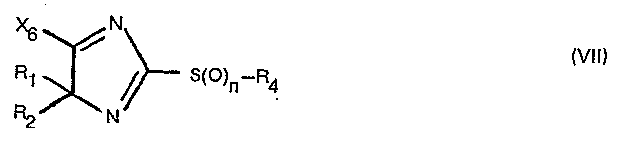 worin X₆ gegebenenfalls durch Phenyl, das seinerseits wie R₁ und R₂ substituiert sein kann, substituiertes Niederalkylthio ist, oder ein Säureadditionssalz davon, umlagert und gewünschtenfalls in einer gemäss der Verfahrensvarianten a) bis f) erhaltenen Verbindung der Formell, worin n für 0 steht, und R₁, R₂, R₃ und R₄ obige Bedeutungen haben, mit der Massgabe, dass in gemäss der Verfahrensvariante a) durch Umsetzung von Verbindungen der Formeln (IU und (lll), worin X gegebenenfalls in Salzform vorliegendes Mercapto und Y reaktionsfähiges verestertes Hydroxy ist, erhaltenen Verbindungen der Formel (I) Heteroarylreste R₁ und/oder R₂ von ein Schwefel- oder Sauerstoffatom aufweisenden 5-gliedrigen oder ein Stickstoffatom aufweisenden 6-gliedrigen Heteroarylreste verschieden sind, wenn R₄ einen durch ein oder zwei Hydroxygruppen, eine Niederalkoxygruppe substituierten oder durch gegebenenfalls wie angegeben substituiertes Phenyl substituierten Niederalkyl- oder unsubstituierten Niederalkinylrest darstellt, die Thiogruppe zur Sulfinyl- oder Sulfonylgruppe oxidiert und/ oder in gemäss einer der Verfahrensvariante a) bis f) erhaltenen Verbindungen, worin R₁. und/oder R₂ mindestens ein Stickstoffatom ⋟N aufweist, dieses N-oxidiert oder in gemäss einer der Verfahrensvariante b) oder f) erhaltenen Verbindungen, worin n für 1 oder 2 steht, die Sulfinyl- oder Sulfonylgruppe zur Thiogruppe und/oder gemäss der Verfahrensvariante a) bis f) erhaltenen Verbindungen, worin R₁ und R₂ ein N-oxidiertes Stickstoffatom aufweist, dieses zu einer Gruppe >N reduziert, eine verfahrensgemäss erhaltene Verbindung der Formel I, worin R₃ Wasserstoff darstellt, N-niederalkyliert, in verfahrensgemäss erhaltenen Verbindungen der Formel I in Reste R₁, R₂ und/oder Phenyl-, Phenoxy-oder Phenylthiogruppen als Bestandteil von R₄ gegebenenfalls zusätzliche C-Substituenten einführt und/oder eine verfahrensgemäss erhaltene freie Verbindung in ein Säureadditionssalz oder ein verfahrensgemäss erhaltenes Säureadditionssalz in die freie Verbindung oder in ein anderes Salz überführt.

30. Verfahren gemäss Anspruch 29 zur Herstellung von Verbindungen der Formel (1), worin R,, R₂, R₃ und R₄ die in Anspruch 29 angegebenen Bedeutungen haben, Heteroarylreste R₁ und/oder R₂ jedoch nicht durch Oxy N-substituiert sind, und n für 0 steht, und ihrer Säureadditionssalze, dadurch gekennzeichnet, dass man
a) Verbindungen der Formeln 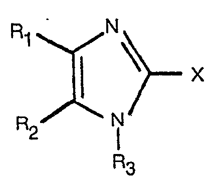 (II) und Y-R₄ (III), worin einer der Reste X und Y gegebenenfalls in Salzform vorliegendes Mercapto und der andere einen gegen veräthertes Mercapto austauschbarer Rest bedeutet, mit der Massgabe, dass in Verbindungen der Formel (₁₁), worin X gegebenenfalls in Salzform vorliegendes Mercapto ist, Heteroarylreste R₁ und/ oder _{R2} von ein Schwefelatom aufweisenden 5-gliedrigen oder ein Stickstoffatom aufweisenden 6- gliedrigen Heteroarylrest oder Reste R₃ von Wasserstoff verschieden sind, wenn in Verbindungen der Formel (III) Y reaktionsfähiges verestertes Hydroxy und R₄ einen in 2-, 3- oder 4-Stellung durch Hydroxy einfach substituierten Niederalkylrest darstellt, miteinander umsetzt oder
c) eine Verbindung der Formel 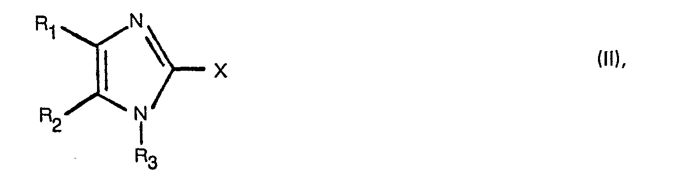 worin X Mercapto darstellt, mit einem gegebenenfalls wie für Niederalkyl R₄ angegeben substituierten Niederalken umsetzt, oder
e) eine Verbindung der Formel 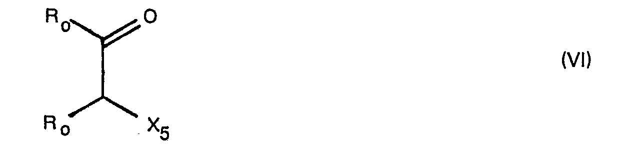 worin der zur Oxogruppe a-ständige Rest Rₒ einen Rest R₁ und der andere Rest Rₐ einen Rest R₂ bedeutet und Xₛ eine reaktionsfähige veresterte Hydroxygruppe bedeutet, mit einem N‾R₃‾S‾R₄‾Iso- thiuroniumsalz umsetzt und gewünschtenfalls eine verfahrensgemäss erhältliche Verbindung der Formel I, worin R₃ für Wasserstoff steht, N-niederalkyliert, in verfahrensgemäss erhältlichen Verbindungen der Formel in Reste R₁, R₂ und/oder Phenyl-, Phenoxy- oder Phenylthiogruppen als Bestandteil von R₄ gegebenenfalls zusätzliche C-Substituenten einführt und/oder eine verfahrensgemäss erhaltene freie Verbindung in ein Säureadditionssalz oder ein verfahrensgemäss erhaltenes Säureadditionssalz in die freie Verbindung überführt.

31. Die nach dem Verfahren des Anspruches 29 oder 30 erhaltenen Verbindungen.

32. Verbindungen gemäss einem der Ansprüche 1-26 als entzündungshemmendes Mittel.

33. Verwendung von Verbindungen gemäss einem der Ansprüche 1-26 zur Herstellung von Arzneimitteln auf nichtchemischem Wege.

## Claims

1. A compound of the formula 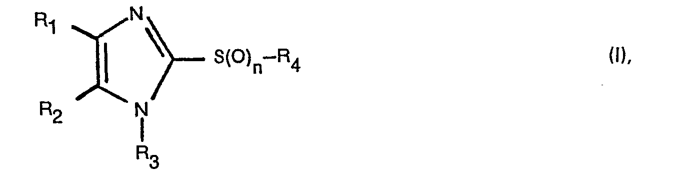 wherein at least one of the radicals R₁ and R₂ is a monocyclic 5-membered heteroaryl group containing one oxygen atom or one sulfur atom, one sulfur atom and one nitrogen atom, or at least two nitrogen atoms, or a monocyclic 6-membered heteroaryl group containing at least one nitrogen atom, which group is linked through a carbon atom and is unsubstituted or C-substituted by lower alkyl, lower alkoxy, halogen, amino, lower alkylamino, di-lower alkylamino, alkyleneamino or 3-aza-, 3-oxa- or 3-thia-alkyleneamino, each containing 5 or 6 ring members, and/or by trifluoromethyl, and/or is N-substituted by lower alkyl, lower alkoxy-lower alkyl, hydroxy-lower alkyl or oxy, and a radical R₁ or R₂ different therefrom is a phenyl group which is unsubstituted or substituted by lower alkyl, lower alkoxy, halogen, amino, lower alkylamino, di-lower alkylamino, alkyleneamino or 3-aza-, 3-oxa- or 3-thiaalkyleneamino, each containing 5 or 6 ring members, or by nitro and/or trifluoromethyl, R₃ is hydrogen or lower alkyl, n is 0, 1 or 2, and R₄ is a lower alkyl, lower alkenyl or lower alkynyl radical which is unsubstituted or substituted by phenyl which is itself unsubstituted or substituted by lower alkyl, lower alkoxy, halogen, amino, lower alkylamino, di-lower alkylamino, alkyleneamino or 3-aza-, 3-oxa- or 3-thiaalkyleneamino, each containing 5 or 6 ring members, or by nitro and/or trifluoromethyl, or is a lower alkyl radical which is substituted in a position higher than the a-position by hydroxy, lower alkoxy, lower alkylthio, or by a phenoxy or phenylthio group which is itself unsubstituted or substituted by lower alkyl, lower alkoxy, halogen, amino, lower alkylamino, di-lower alkylamirio, alkyleneamino or 3-aza-, 3-oxa- or 3-thiaalkyleneamino, each containing 5 or 6 ring members, or by nitro and/or trifluoromethyl, with the proviso that heteroaryl radicals R₁ and/or R₂ are different from 5-membered heteroaryl radicals containing one sulfur atom and from 6-membered heteroaryl radicals containing one nitrogen atom, or radicals R₃ are different from hydrogen, if R₄ is lower alkyl substituted in the 2-, 3- or 4-position by a hydroxyl group, and with the further proviso that, in a compound of the formula I, in which n is 1 or 2, heteroaryl radicals R₁ and/or R₂ differ from 5-membered heteroaryl radicals containing one sulfur atom or one oxygen atom, and from 6-membered heteroaryl radicals containing one nitrogen atom, if R₄ is lower alkyl substituted by one or two hydroxyl groups, a lower alkoxy group or by phenyl which is unsubstituted or substituted as indicated above, or is unsubstituted lower alkynyl, the radicals qualified by "lower" containing up to and including 7 carbon atoms, or an acid addition salt thereof.

2. A compound according to claim 1, wherein at least one of the radicals R₁ and R₂ is a monocyclic 5-membered heteroaryl group containing one oxygen atom or one sulfur atom, one sulfur atom and one nitrogen atom, or at least two nitrogen atoms, or a monocyclic 6-membered heteroaryl group containing at least one nitrogen atom, which group is linked through a carbon atom and is unsubstituted or C-substituted by lower alkyl, lower alkoxy, halogen, amino, lower alkylamino, di-lower alkylamino, alkyleneamino or 3-aza-, 3-oxa- or 3-thiaalkyleneamino, each containing 5 or 6 ring members, and/or by trifluoromethyl, and/or is N-substituted by lower alkyl, lower alkoxy-lower alkyl, hydroxy-lower alkyl, and a radical R₁ or R₂ different therefrom is a phenyl group which is unsubstituted or substituted by lower alkyl, lower alkoxy, halogen, amino, lower alkylamino, di-lower alkylamino, alkyleneamino or 3-aza-, 3-oxa- or 3-thiaalkyleneamino, each containing 5 or 6 ring members, or by nitro and/or trifluoromethyl, R₃ is hydrogen or lower alkyl, n is 0, and R₄ is a lower alkyl, lower alkenyl or lower alkynyl radical which is unsubstituted or substituted by phenyl which is itself unsubstituted or substituted by lower alkyl, lower alkoxy, halogen, amino, lower alkylamino, di-lower alkylamino, alkyleneamino or 3-aza-, 3-oxa- or 3-thiaalkyleneamino, each containing 5 or 6 ring members, or by nitro and/or trifluoromethyl, or is a lower alkyl radical which is substituted in a position higher than the a-position by hydroxy, lower alkoxy, lower alkylthio, or by a phenoxy or phenylthio group which is itself unsubstituted or substituted by lower alkyl, lower alkoxy, halogen, amino, lower alkylamino, di-lower alkylamino, alkyleneamino or 3-aza-, 3-oxa- or. 3-thiaatkyteneamino, each containing 5 or 6 ring members, or by nitro and/or trifluoromethyl, with the proviso that heteroaryl radicals R₁ and/or R₂ are different from 5-membered heteroaryl radicals containing one sulfur atom and from 6-membered heteroaryl radicals containing one nitrogen atom, or radicals R₃ are different from hydrogen, if R₄ is lower alkyl substituted in the 2-, 3- or 4-position by a hydroxyl group, or an acid addition salt thereof.

3. A compound according to claim 1, wherein at least one of the radicals R₁ and R₂ is furyl, thienyl, thiazolyl, imidazolyl, triazolyl, tetrazolyl, pyridyl, pyrimidinyl or triazinyl, and the ring carbon atoms of said radicals R₁ and R₂ may be substituted by lower alkyl, lower alkoxy, halogen having an atomic number of up to and including 35, trifluoromethyl, amino, lower alkylamino and/or alkyleneamino or 3-aza, 3-oxa- or 3-thiaalkyleneamino, each containing 5 or 6 ring members, and the ring nitrogen atoms of pyrimidinyl, triazinyl or pyridyl radicals may also be substituted by oxy, and a radical R₁ or R₂ different therefrom is phenyl which may be substituted by lower alkyl, lower alkoxy, halogen having an atomic number of up to and including 35, trifluoromethyl, nitro, amino, lower alkylamino and/or alkyleneamino or 3-aza, 3-oxa- or 3-thiaalkyleneamino, each containing 5 or 6 ring members, R₃ is hydrogen or lower alkyl, n is 0, 1 or 2, and R₄ is a lower alkyl, lower alkenyl or lower alkynyl radical which is unsubstituted or substituted by lower alkyl, lower alkoxy, halogen having an atomic number of up to and including 35, trifluoromethyl, nitro, amino, lower alkylamino and/or alkyleneamino or 3-aza-, 3-oxa- or 3-thiaalkyleneamino, each containing 5 or 6 ring members, or is a tower alkyl radical which is substituted in a position higher than the a-position by hydroxy, lower alkoxy, lower alkylthio, or by a phenoxy or pₕₑₙyₗthᵢₒ group which is itself unsubstituted or substituted by lower alkyl, lower alkoxy, halogen having an atomic number of up to and including 35, trifluoromethyl, nitro, amino, lower alkyleneamino and/or alkyleneamino or 3-aza-, 3-oxa- or 3-thiaalkyleneamino, each containing 5 or 6 ring members, or an acid addition salt thereof.

4. A compound according to claim 2, wherein at least one of the radicals R₁ and R₂ is furyl, thienyl, thiazolyl, imidazolyl, triazolyl, tetrazolyl, pyridyl, pyrimidinyl or triazinyl, and the ring carbon atoms of said radicals R₁ and R₂ may be substituted by lower alkyl, lower alkoxy, halogen having an atomic number of up to and including 35, trifluoromethyl, amino, lower alkylamino and/or alkyleneamino or 3-aza, 3-oxa- or 3-thiaalkyleneamino, each containing 5 or 6 ring members, and a radical R₁ or R₂ different therefrom is phenyl which may be substituted by lower alkyl, lower alkoxy, halogen having an atomic number of up to and including 35, trifluoromethyl, nitro, amino, lower alkylamino and/or alkyleneamino or 3-aza, 3-oxa- or 3-thiaalkyleneamino, each containing 5 or 6 ring members, R₃ is hydrogen or lower alkyl, n is 0, and R₄ is a lower alkyl, lower alkenyl or lower alkynyl radical which is unsubstituted or substituted by lower alkyl, lower alkoxy, halogen having an atomic number of up to and including 35, trifluoromethyl, nitro, amino, lower alkylamino and/or alkyleneamino or 3-aza-, 3-oxa-or 3-thiaalkyleneamino, each containing 5 or 6 ring members, or is a lower alkyl radical which is substituted in a position higher than the a-position by hydroxy, lower alkoxy, lower alkylthio, or by a phenoxy or phenylthio group which is itself unsubstituted or substituted by lower alkyl, lower alkoxy, halogen having an atomic number of up to and including 35, trifluoromethyl, nitro, amino, lower alkyleneamino and/or alkyleneamino or 3-aza-, 3-oxa- or 3-thiaalkyleneamino, each containing 5 or 6 ring members, or an acid addition salt thereof.

5. A compound according to claim 2, in which one of the radicals R₁ and R₂ is pyridyl and the other is phenyl, and these radicals independently of each other may be substituted by lower alkyl of 1 to 4 carbon atoms, lower alkoxy of 1 to 4 carbon atoms, halogen having an atomic number of up to and including 35 and/or N,N-di-lower alkylamino containing 1 to 4 carbon atoms in each alkyl moiety, R₃ is hydrogen or lower alkyl having 1 to 4 C atoms, n is 0 and R₄ is lower alkyl of 1 to 4 carbon atoms, lower alkenyl of 2 to 4 carbon atoms, phenyl-lower alkyl which contains 1 to 4 carbon atoms in the alkyl moiety and is unsubstituted or substituted by lower alkyl of 1 to 4 carbon atoms, lower alkoxy of 1 to 4 carbon atoms and/or halogen having an atomic number of up to and including 35, mono- or dihydroxy- lower alkyl which contains 2 to 4 carbon atoms and carries the hydroxyl group or groups in a position higher than the a-position, or lower alkoxy- or lower alkylthio-Iower alkyl, which contains 1 to 4 carbon atoms in the alkoxy or alkylthio moiety and 2 to 4 carbon atoms in the alkyl moiety, and carries the alkoxy or alkylthio group in a position higher than the a-position, or an acid addition salt thereof.

6. A compound according to claim 1, wherein one of the radicals R₁ and R₂ is unsubstituted pyridyl or thienyl and the other is phenyl which is unsubstituted or substituted by lower alkyl of 1 to 4 carbon atoms, lower alkoxy of 1 to 4 carbon atoms and/or halogen having an atomic number of up to and including 35, R₃ is hydrogen or lower alkyl of 1 to 4 carbon atoms, n is 0, 1 or 2, and R₄ is lower alkyl of 1 to 4 carbon atoms, or an acid addition salt thereof.

7. A compound according to claim 2, wherein one of the radicals R₁ and R₂ is unsubstituted pyridyl and the other is phenyl which is unsubstituted or substituted by lower alkyl of 1 to 4 carbon atoms, lower alkoxy of 1 to 4 carbon atoms or halogen, R₃ is hydrogen or lower alkyl of 1 to 4 carbon atoms, n is 0, and R₄ is lower alkyl of 1 to 4 carbon atoms or hydroxy-lower alkyl of 2 to 4 carbon atoms which carries the hydroxyl group in a position higher than the a-position, or an acid addition salt thereof.

8. A compound according to claim 1, wherein one of the radicals R1 and R₂ is pyridyl and the other is phenyl, R₃ is hydrogen, n is 0 or 1, and R₄ is lower alkyl of 1 to 4 carbon atoms, or an acid addition salt thereof.

9. A compound according to any one of claims 1 to 8 as antiinflammatory or antinociceptive agent.

10. 2-Ethylthio-4(5)-phenyl-5(4)-(3-pyridyl)-imidazole or an acid addition salt thereof.

11. 2-Ethanesulfinyl-4(5)-phenyl-5(4)-(3-pyridyl)-imidazole or an acid addition salt thereof.

12. 2-Ethylthio-4(5)-(p-fluorophenyl-5(4)-(2-thienyl)-imidazole or an acid addition salt thereof.

13. 2-(2-Hydroxyethylthio)-4(5)-phenyl-5(4)-(3-pyridyl)-imidazole or an acid addition salt thereof.

14. 2-Methylthio-4(5)-phenyl-5(4)-(3-pyridyl)-imidazole or an acid addition salt thereof.

15. 2-(2-Methylthioethylthio)-4(5)-phenyl-5(4)-(3-pyridyl)-imidazole or an acid addition salt thereof.

16. 2-(2-Methoxyethylthio)-4(5)-phenyl-5(4)-(3-pyridyl)-imidazole or an acid addition salt thereof.

17. 2-Propylthio-4(5)-phenyl-5(4)-(3-pyridyl)-imidazole or an acid addition salt thereof.

18. 2-lsopropylthio-4(5)-phenyl-5(4)-(3-pyridyl)-imidazole or an acid addition salt thereof.

19. 2-Ethanesulfonyl-4(5)-phenyl-5(4)-(3-pyridyl)-imidazole or an acid addition salt thereof.

20. 2-Ethanesulfinyl-4(5)-(p-fluorophenyl)-(2-thienyl)-imidazole or an acid addition salt thereof.

21. 2-Ethanesulfonyl-4(5)-(p-fluorophenyl)-(2-thienyl)-imidazole or an acid addition salt thereof.

22. 2-Ethylthio-4(5)-bis-(2-thienyl)-imidazole or an acid addition salt thereof.

23. A compound of the formula 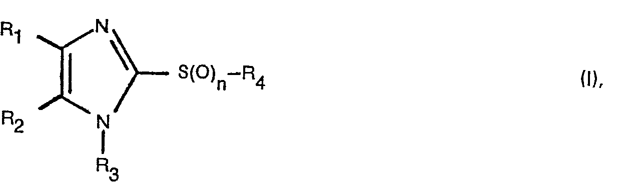 wherein at least one of the radicals R₁ and R₂ is a monocyclic 5-membered heteroaryl group containing one oxygen atom or one sulfur atom, one sulfur atom and one nitrogen atom, or at least two nitrogen atoms, or a monocyclic 6-membered heteroaryl group containing at least one nitrogen atom, which group is linked through a carbon atom and is unsubstituted or C-substituted by lower alkyl, lower alkoxy, halogen, amino, lower alkylamino, di-lower alkylamino, alkyleneamino or 3-aza-, 3-oxa- or 3-thiaalkyleneamino, each containing 5 or 6 ring members, and/or by trifluoromethyl, and/or is N-substituted by lower alkyl, lower alkoxy-lower alkyl, hydroxy-lower alkyl or oxy, and a radical R₁ or R₂ different therefrom is a phenyl group which is unsubstituted or substituted by lower alkyl, lower alkoxy, halogen, amino, lower alkylamino, di-lower alkylamino, alkyleneamino or 3-aza, 3-oxa- or 3-thiaalkyleneamino, each containing 5 or 6 ring members, or by nitro and/or trifluoromethyl, R₃ is hydrogen or lower alkyl, n is 0, 1 or 2, and R₄ is a lower alkyl, lower alkenyl or lower alkynyl radical which is unsubstituted or substituted by phenyl which is itself unsubstituted or substituted by lower alkyl, lower alkoxy, halogen, amino, lower alkylamino, di-lower alkylamino, alkyleneamino or 3-aza, 3-oxa- or 3-thiaalkyleneamino, each containing 5 or 6 ring members, or by nitro and/or trifluoromethyl, or is a lower alkyl radical which is substituted in a position higher than the a-position by hydroxy, lower alkoxy, lower alkylthio, or by a phenoxy or phenylthio group which is itself unsubstituted or substituted by lower alkyl, lower alkoxy, halogen, amino, lower alkylamino, di-lower alkylamino, alkyleneamino or 3-aza-, 3-oxa- or 3-thiaalkyleneamino, each containing 5 or 6 ring members, or by nitro and/or trifluoromethyl, with the proviso that, in a compound of the formula I, in which n is 1 or 2, heteroaryl radicals R₁ and/or R₂ differ from 5-membered heteroaryl radicals containing one sulfur atom or one oxygen atom, and from 6-membered heteroaryl radicals containing one nitrogen atom, if R₄ is lower alkyl substituted by one or two hydroxyl groups, by a lower alkoxy group or by phenyl which is unsubstituted or substituted as indicated above, or is an unsubstituted lower alkynyl radical, the radicals qualified by "lower" containing up to and including 7 carbon atoms, or a pharmaceutically acceptable acid addition salt thereof, for the treatment of inflammatory conditions.

24. A compound of the formula 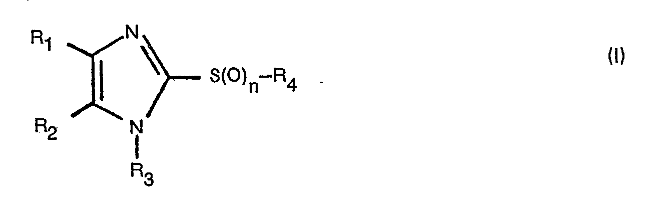 wherein at least one of the radicals R₁ and R₂ is a monocyclic 5-membered heteroaryl group containing one oxygen atom or one sulfur atom, one sulfur atom and one nitrogen atom, or at least two nitrogen atoms, or a monocyclic 6-membered heteroaryl group containing at least one nitrogen atom, which group is linked through a carbon atom and is unsubstituted or C-substituted by lower alkyl, lower alkoxy, halogen, amino, lower alkylamino, di-lower alkylamino, alkyleneamino or 3-aza-, 3-oxa- or 3-thiaalkyleneamino, each containing 5 or 6 ring members, and/or by trifluoromethyl, and/or is N-substituted by lower alkyl, lower alkoxy-lower alkyl, hydroxy-lower alkyl, and a radical R₁ or R₂ different therefrom is a phenyl group which is unsubstituted or substituted by lower alkyl, lower alkoxy, halogen, amino, lower alkylamino, di-lower alkylamino, alkyleneamino or 3-aza-, 3-oxa- or 3-thiaalkyleneamino, each containing 5 or 6 ring members, or by nitro and/or trifluoromethyl, R₃ is hydrogen or lower alkyl, n is 0, 1 or 2, and R₄ is a lower alkyl, lower alkenyl or lower alkynyl radical which is unsubstituted or substituted by phenyl which is itself unsubstituted or substituted by lower alkyl, lower alkoxy, halogen, amino, lower alkylamino, di-lower alkylamino, alkyleneamino or 3-aza-, 3-oxa- or 3-thiaalkyleneamino, each containing 5 or 6 ring members, or by nitro and/or trifluoromethyl, or is a lower alkyl radical which is substituted in a position higher than the a-position by hydroxy, lower alkoxy, lower alkylthio, or by a phenoxy or phenylthio group which is itself unsubstituted or substituted by lower alkyl, lower alkoxy, halogen, amino, lower alkylamino, di-lower alkylamino, alkyleneamino or 3-aza-, 3-oxa- or 3-thiaalkyleneamino, each containing 5 or 6 ring members, or by nitro and/or trifluoromethyl, the radicals qualified by "lower" containing up to and including 7 carbon atoms, or a pharmaceutically acceptable acid addition salt thereof, for the treatment of inflammatory conditions.

25. A compound according to any one of claims 2, 4, 5, 7, 10, 13-18 and 22, for the treatment of inflammatory conditions. 1

26. A compound according to any one of claims 1, 3, 6, 8, 9 and 19-21, for the treatment of inflammatory conditions.

27. A pharmaceutical preparation containing a compound according to any one of claims 2, 4, 5, 7, 10, 13-18, 22 and 25, together with conventional pharmaceutical excipients.

28. A pharmaceutical preparation containing a compound according to any one of claims 1, 3, 6, 8, 9, 19-21, 23 and 26, together with conventional pharmaceutical excipients.

29. A process for the production of a compound of the formula 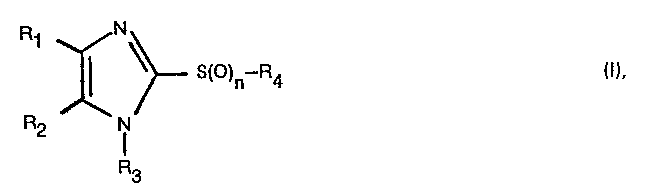 wherein at least one of the radicals R₁ and R₂ is a monocyclic 5-membered heteroaryl group containing one oxygen atom or one sulfur atom, one sulfur atom and one nitrogen atom, or at least two nitrogen atoms, or a monocyclic 6-membered heteroaryl group containing at least one nitrogen atom, which group is linked through a carbon atom and is unsubstituted or C-substituted by lower alkyl, lower alkoxy, halogen, amino, lower alkylamino, di-lower alkylamino, alkyleneamino or 3-aza-, 3-oxa- or 3-thia-alkyleneamino, each containing 5 or 6 ring members, and/or by trifluoromethyl, and/or is N-substituted by lower alkyl, lower alkoxy-lower alkyl, hydroxy-lower alkyl and a radical R₁ or R₂ different therefrom is a phenyl group which is unsubstituted or substituted by lower alkyl, lower alkoxy, halogen, amino, lower alkylamino, di-lower alkylamino, alkyleneamino or 3-aza-, 3-oxa- or 3-thiaalkyleneamino, each containing 5 or 6 ring members, or by nitro and/or trifluoromethyl, R₃ is hydrogen or lower alkyl, n is 0, and R4 is a lower alkyl, lower alkenyl or lower alkynyl radical which is unsubstituted or substituted by phenyl which is itself unsubstituted or substituted by lower alkyl, lower alkoxy, halogen, amino, lower alkylamino, di-lower alkylamino, alkyleneamino or 3-aza-, 3-oxa- or 3-thiaalkyleneamino, each containing 5 or 6 ring members, or by nitro and/or trifluoromethyl, or is a lower alkyl radical which is substituted in a position higher than the a-position by hydroxy, lower alkoxy, lower alkylthio, or by a phenoxy or phenylthio group which is itself unsubstituted or substituted by lower alkyl, lower alkoxy, halogen, amino, lower alkylamino, di-lower alkylamino, alkyleneamino or 3-aza-, 3-oxa- or 3-thiaalkyleneamino, each containing 5 or 6 ring members, or by nitro and/or trifluoromethyl, the radicals qualified by "lower" containing up to and including 7 carbon atoms, or an acid addition salt thereof, which process comprises
a) reacting compounds of the formulae 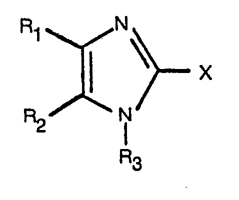 (II) and Y-R₄ (III), wherein one of the radicals X and Y is mercapto which may be in salt form and the other is a radical which is replaceable by etherified mercapto, with the proviso that, in compounds of the formula (III), in which X is mercapto which may be in salt form, heteroaryl radicals R₁ and/or R₂ differ from 5- membered heteroaryl radicals containing a sulfur atom and from 6-membered heteroaryl radicals containing a nitrogen atom, or radicals R₃ differ from hydrogen, if in compounds of the formula (III) Y is reactive esterified hydroxy and R₄ is lower alkyl monosubstituted in the 2-, 3- or 4-position by hydroxy, with one another, or
b) reacting compounds of the formulae 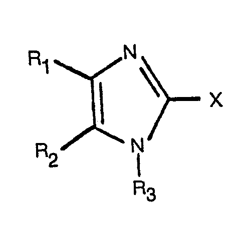 (11) and Y-R₄ (III), wherein one of the radicals X and Y is a group of the formula ‾S(O)ₙ‾Y₁, wherein Y₁ is a reactive esterified hydroxyl group or, if n is 0, an etherified mercapto group, or if n is 1 or 2, an etherified hydroxyl group, and the other is a metal radical Y₂, with one another, or
c) reacting a compound of the formula 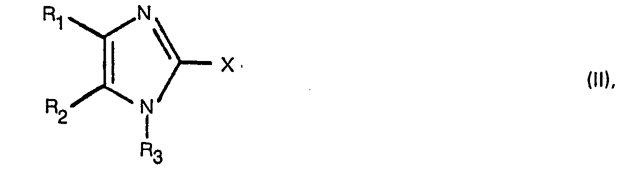 wherein X is the mercapto group, with a lower alkene which is unsubstituted or substituted as indicated for lower alkyl R₄, or
d) reacting a compound of the formula 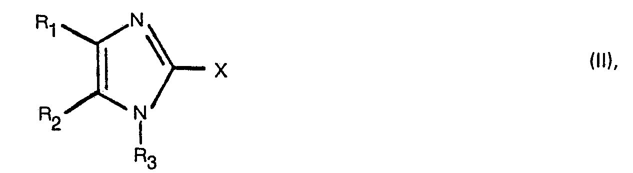 wherein X is a sulfinic or sulfonic acid group in salt form, with a compound of the formula 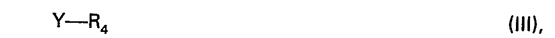 wherein Y is a reactive esterified hydroxyl group, or
e) reacting a compound of the formula 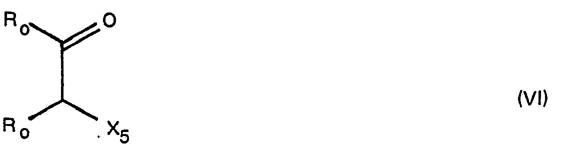 wherein one of the radicals Ro is a group R₁ and the other is a group R₂ and X₅ is a reactive esterified hydroxyl group, or an acid addition salt thereof, with a N‾R₃‾S‾R₄ isothiourea, or
f) rearranging a compound of the formula 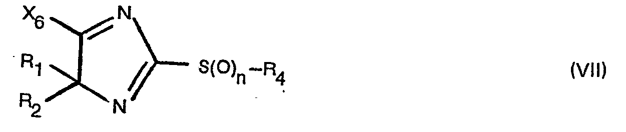 wherein X₆ is lower alkylthio which is unsubstituted or substituted by phenyl which may itself be substituted as for R₁ and R₂, or an acid addition salt thereof, and, if desired, in a compound of the formula I obtained by one of process variants a) to f) and in which n is 0, and R₁, R₂, R₃ and R₄ are as defined above, with the proviso that, in compounds of the formula I obtained by process variant a) by reacting compounds of the formulae (11) and (II), in which X is mercapto which may be in salt form and Y is reactive esterified hydroxy, heteroaryl radicals R₁ and/or R₂ differ from 5-membered heteroaryl radicals containing a sulfur atom or an oxygen atom, or from 6-membered heteroaryl radicals containing a nitrogen atom, if R₄ is a lower alkyl radical which is substituted by one or two hydroxyl groups, a lower alkoxy group or by phenyl which is unsubstituted or substituted as indicated, or is an unsubstituted lower alkynyl radical, oxidising the thio group to the sulfinyl or sulfonyl group, and/or in a compound obtained by one of process variants a) to f), wherein R₁ and/or R₂ contain at least one nitrogen atom > N, N-oxidising said nitrogen atom, or in a compound obtained by one of process variants b) or f), in which n is 1 or 2, reducing the sulfinyl or sulfonyl group to the thio group, and/or in a compound obtained by one of process variants a) to f), in which R₁ and R₂ contain a N-oxidised nitrogen atom, reducing said nitrogen atom to a group > N, N-lower alkylating a compound of the formula I, in which R₃ is hydrogen, or in a compound of the formula I optionally introducing additional C-substituents into radicals R₁, R₂ and/or into phenyl, phenoxy or phenylthio groups as constituents of R₄, and/or converting a free compound into an acid addition salt or an acid addition salt into the free compound or into another salt.

30. A process according to claim 29 for the production of a compound of the formula (I), in which R₁, R₂, R₃ and R₄ are as defined in claim 29, but heteroaryl radicals R₁ and/or R₂ are not N-substituted by oxy, and n is 0, or an acid addition salt thereof, which process comprises
a) reacting compounds of the formulae 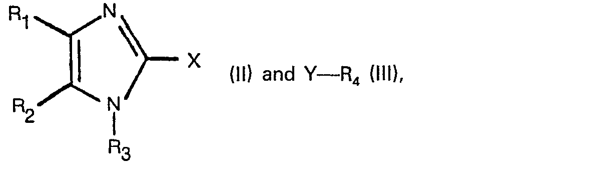 wherein one of the radicals X and Y is mercapto which may be in salt form and the other is a radical which is replaceable by etherified mercapto, with the proviso that, in compounds of the formula (II), in which X is mercapto which may be in salt form, heteroaryl radicals R₁ and/or R₂ differ from 5.. membered heteroaryl radicals containing a sulfur atom, and from 6-membered heteroaryl radicals containing a nitrogen atom, or radicals R₃ are different from hydrogen, if in compounds of the formula (III) Y is reactive esterified hydroxy and R₄ is lower alkyl mono-substituted in the 2-, 3- or 4-position by hydroxy, with one another, or
c) reacting a compound of the formula 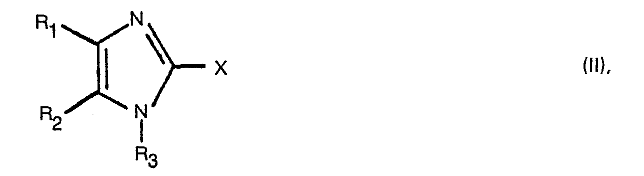 wherein X is the mercapto group, with a lower alkene which is unsubstituted or substituted as indicated for lower alkyl R₄, or
e) reacting a compound of the formula 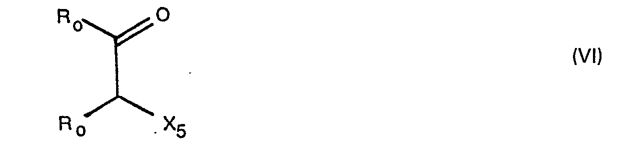 wherein the radical Ro α-oriented to the oxo group is a radical R₁ and the other radical Rₒ is a radical R₂ and Xₛ is a reactive esterified hydroxyl group, with a N‾R₃‾S‾R₄ isothiuronium salt and, if desired, N-lower alkylating a compound of the formula I in which R₃ is hydrogen, in compounds of the formula I optionally introducing additional C-substituents into radicals R₁, R₂ and/or into phenyl, phenoxy or phenylthio groups as constituents of R₄, and/or converting a free compound into an acid addition salt or an acid addition salt into the free compound.

31. A compound obtained by the process as claimed in either of claim 29 or claim 30.

32. A compound as claimed in any one of claims 1 to 26 as antiinflammatory agent.

33. Use of a compound as claimed in any one of claims 1 to 26 for the preparation of medicaments by a non-chemical route.

## Revendications

1. Composés de formule 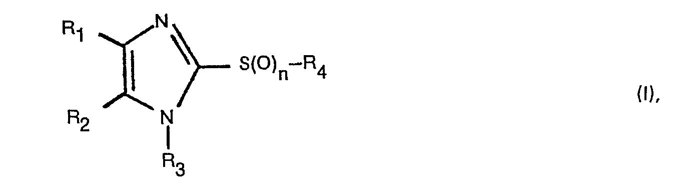 dans laquelle l'un au moins des symboles R₁ et R₂ représente un groupe hétéroaryle monocyclique relié par l'intermédiaire d'un atome de carbone, à 5 chaînons contenant un atome d'oxygène ou de soufre, un atome de soufre et un atome d'azote ou au moins deux atomes d'azote ou à 6 chaînons contenant au moins un atome d'azote, non substitué ou substitué sur le carbone par des groupes alkyles inférieurs, alcoxy inférieurs, des halogènes, des groupes amino, alkylamino inférieurs, di-(alkyle inférieur)-amino, alkylène ou 3-aza-, 3-oxa- ou 3-thia-alkylène-amino contenant chacun 5 ou 6 chaînons cycliques et/ou trifluorométhyle, et/ou substitué à l'azote par des groupes alkyles inférieurs, (alcoxy inférieur)-alkyles inférieurs, hydroxyalkyles inférieurs ou oxy, et un symbole R₁ ou R₂ différent du précédent représente un groupe phényle non substitué ou substitué par des groupes alkyles inférieurs, alcoxy inférieurs, des halogènes, des groupes amino, alkylamino inférieurs, di-(alkyle inférieur)-amino, alkylène-amino ou 3-aza-, 3-oxa- ou 3-thia-alkylène-amino contenant chacun 5 ou chaînons cycliques, nitro et/ou tri-fluorométhyle, R₃ représente l'hydrogène ou un groupe alkyle inférieur, n est égal à 0,1 ou 2 et R₄ représente un groupe alkyle inférieur, alcényle inférieur ou alcynyle inférieur non substitué ou substitué par un groupe phényle non substitué ou substitué par des groupes alkyles inférieurs, alcoxy inférieurs, des halogènes, des groupes amino, alkylamino inférieurs, di-(alkyle inférieur)-amino, alkylène-amino ou 3-aza-, 3-oxa- ou 3-thia-alkylène-amino contenant chacun 5 ou 6 chaînons cycliques, nitro et/ou trifluorométhyle, ou un groupe alkyle inférieur substitué en position supérieure à la position alpha par des groupes hydroxy, alcoxy inférieurs, alkylthio inférieurs, un groupe phénoxy ou phénylthio non substitué ou substitué par des groupes alkyles inférieurs, alcoxy inférieurs, des halogènes, des groupes amino, alkylamino inférieurs, di-(alkyle inférieur)-amino, alkylène-amino ou 3-aza-, 3-oxa- ou 3-thia-alkylène-amino contenant chacun 5 ou 6 chaînons cycliques, nitro et/ou trifluorométhyle, étant spécifié que les groupes hétéroaryles R₁ et/ou R₂ diffèrent de groupes hétéroaryles à chaînons portant un atome de soufre et de groupes hétéroaryles à chaînons portant un atome d'azote, ou les restes R₃ diffèrent de l'hydrogène lorsque R₄ représente ungroupe alkyle inférieur substitué par un groupe hydroxy en position 2, 3 ou 4, et étant en outre spécifié que dans les composés de formule dans laquelle n est égal à 1 ou 2, les groupes hétéroaryles R₁ et/ou R₂ diffèrent de groupes hétéroaryles à 5 chaînons portant un atome de soufre ou d'oxygène et de groupes hétéroaryles à 6 chaînons contenant un atome d'azote lorsque R₄ représente un groupe alkyle inférieur substitué par un ou deux groupes hydroxy, un groupe alcoxy inférieur, ou par un groupe phényle éventuellement substitué comme indiqué ci-dessus, ou un groupe alcynyle inférieur non substitué, les restes "inférieurs" contenant jusqu'à 7 atomes de carbone inclus, et leurs sels formés par addition avec des acides.

2. Composés selon la revendication 1, dans lesquels l'un au moins des symboles R₁ et R₂ représente un groupe hétéroaryle monocyclique, relié par l'intermédiaire d'un atome de carbone, à 5 chaînons contenant un atome d'oxygène ou de soufre, un atome de soufre et un atome d'azote ou au moins deux atomes d'azote, ou à 6 chaînons contenant au moins un atome d'azote, non substitué ou substitué sur le carbone par des groupes alkyles inférieurs, alcoxy inférieurs, des halogènes, des groupes amino, alkylamino inférieurs, di-(alkyle inférieur)-amino, alkylène- ou 5-aza-, 3-oxa- ou 3-thia-alkylène-amino contenant chacun 5 ou 6 chaînons cycliques et/ou trifluorométhyle, et/ou substitué sur l'azote par des groupes alkyles inférieurs, (alcoxy inférieur)-alkyles inférieurs ou hydroxyalkyles inférieurs, et un reste R₁ ou R₂ différent du précédent est un groupe phényle non substitué ou substitué par des groupes alkyles inférieurs, alcoxy inférieurs, des halogènes, des groupes amino, alkylamino inférieurs, di-(alkyle inférieur)-amino, alkylène-amino ou 3-aza-, 3-oxa- ou 3-thia-alkylène-amino contenant chacun 5 ou 6 chaînons cycliques, nitro et/ou trifluorométhyle, R₃ représente l'hydrogène ou un groupe alkyle inférieur, n est égal à 0 et R₄ représente un groupe alkyle inférieur, alcényle inférieur ou alcynyle inférieur non substitué ou substitué par un groupe phényle non substitué ou substitué par des groupes alkyles inférieurs, alcoxy inférieurs, des halogènes, des groupes amino, alkylamino inférieurs, di-(alkyle inférieur)-amino, alkylène-amino ou 3-aza, 3-oxa- ou 3-thia-alkylène-amino contenant chacun 5 ou 6 chaînons cycliques, nitro et/ou trifluorométhyle, ou bien un groupe alkyle inférieur substitué en position supérieure à la position alpha par des groupes hydroxy, alcoxy inférieurs, alkylthio inférieurs, un groupe phénoxy ou phénylthio non substitué ou substitué par des groupes alkyles inférieurs, alcoxy inférieurs, des halogènes, des groupes amino, alkylamino inférieurs, di-(alkyle inférieur)-amino, alkylèneamino ou 3-aza-, 3-oxa- ou 3-thia-alkylène-amino contenant chacun 5 ou 6 chaînons cycliques, nitro et/ou trifluorométhyle, étant spécifié que les groupes hétéroaryles R₁ et R₂ diffèrent de groupes hétéroaryles à 5 chaînons contenant un atome de soufre et de groupes hétéroaryles à 6 chaînons contenant un atome d'azote lorsque R₄ représente un groupe alkyle inférieur substitué par un groupe hydroxy en position 2, 3 ou 4, et leurs sels formés par addition avec des acides.

3. Composés selon la revendication 1, dans lesquels l'un au moins des symboles R₁ et R₂ représente un groupe furyle, thiényle, thiazolyle, imidazolyle, triazolyle, tétrazolyle, pyridyle, pyrimidinyle ou triazinyle, les atomes de carbone cycliques des restes R₁ et R₂ en question pouvant être substitués par des groupes alkyles inférieurs, alcoxy inférieurs, des halogènes de nombre atomique allant jusqu'à 35, des groupes trifluorométhyle, amino, alkylamino inférieurs et/ou alkylène- ou 3-aza-, 3-oxa- ou 3-thia-alkylène-amino contenant chacun 5 ou 6 chaînons cycliques, les atomes d'azote cycliques des restes pyrimidinyle, triazinyle ou pyridyle pouvant en outre porter un substituant oxy, et un symbole R₁ ou R₂ différent du précédent représente un groupe phényle qui peut être substitué par des groupes alkyles inférieurs, alcoxy inférieurs, des halogènes de nombre atomique allant jusqu'à 35, des groupes trifluorométhyle, nitro, amino, alkylamino inférieurs et/ou alkylène- ou 3-aza-, 3-oxa- ou 3-thia-alkylène-amino contenant chacun 5 ou 6 chaînons cycliques, R₃ représente l'hydrogène ou un groupe alkyle inférieur, n est égal à 0, 1 ou 2 et R₄ représente un groupe alkyle inférieur, alcényle inférieur, ou alcynyle inférieur non substitué ou substitué par un groupe phényle éventuellement substitué par des groupes alkyles inférieurs, alcoxy inférieurs, des halogènes de nombre atomique allant jusqu'à 35, des groupes trifluorométhyle, nitro, amino, alkylamino inférieurs, et/ou alkylène- ou 3-aza-, 3-oxa- ou 3-thia-alkylène-amino contenant chacun 5 ou 6 chaînons cycliques, ou un groupe alkyle inférieur substitué dans une position supérieure à la position alpha par des groupes hydroxy, alcoxy inférieurs, alkylthio inférieurs ou un groupe phénoxy ou phénylthio éventuellement substitué par des groupes alkyles inférieurs, alcoxy inférieurs, des halogènes de nombre atomique allant jusqu'à 35, des groupes trifluorométhyle, nitro, amino, alkylamino inférieurs et/ou alkylène- ou 3-aza-, 3-oxa- ou 3-thia-alkylène-amino contenant chacun 5 ou 6 chaînons cycliques, et leurs sels formés par addition avec des acides.

4. Composés selon la revendication 2, dans lesquels l'un au moins des symboles R₁ et R₂ représente un groupe furyle, thiényle, thiazolyle, imidazolyle, triazolyle, tétrazolyle, pyridyle; pyrimidinyle ou triazinyle, les atomes de carbone cycliques des restes R₁ et R₂ en question pouvant être substitués par des groupes alkyles inférieurs, alcoxy inférieurs, des halogènes de nombre atomique allant jusqu'à 35, des groupes trifluorométhyle, amino, alkylamino inférieurs et/ou alkylène- ou 3-aza-, 3-oxa- ou 3-thia-alkylène-amino contenant chacun 5 ou 6 chaînons cycliques, et un symbole R₂ ou R₃ différent du précédent représente un groupe phényle qui peut être substitué par des groupes alkyles inférieurs, alcoxy inférieurs, des halogènes de nombre atomique allant jusqu'à 35 inclus, des groupes trifluorométhyle, nitro, amino, alkylamino inférieurs et/ou alkylène- ou 3-aza-, 3-oxa- ou 3-thia-alkylène-amino contenant chacun 5 ou 6 chaînons cycliques, R₃ représente l'hydrogène ou un groupe alkyle inférieur, n est égal à 0 et R₄ représente un groupe alkyle inférieur, alcényle inférieur, ou alcynyle inférieur non substitué ou substitué par un groupe phényle éventuellement substitué par des groupes alkyles inférieurs, alcoxy inférieurs, des halogènes de nombre atomique allant jusqu'à 35 inclus, des groupes trifluorométhyle, nitro, amino, alkylamino inférieurs et/ou alkylène- ou 3-aza-, 3-oxa- ou 3-thia-alkylène-amino contenant chacun 5 ou 6 chaînons cycliques, ou un groupe alkyle inférieur substitué dans une position supérieure à la position alpha par des groupes hydroxy, alcoxy inférieurs, alkylthio inférieurs ou un groupe phénoxy ou phénylthio éventuellement substitué par des groupes alkyles inférieurs, alcoxy inférieurs, des halogènes de nombre atomique allant jusqu'à 35 inclus, des groupes trifluorométhyle, nitro, amino, alkylamino inférieurs et/ou alkylène- ou 3-aza-, 3-oxa- ou 3-thia-alkylène-amino contenant chacun 5 ou 6 chaînons cycliques, et leurs sels formés par addition avec des acides.

5. Composés selon la revendication 2, dans lesquels l'un des symboles R₁ et R₂ représente un groupe pyridyle et l'autre un groupe phényle, ces groupes pouvant être substitués indépendamment l'un de l'autre par des groupes alkyles inférieurs en Cl-C4, alcoxy inférieurs an Cl-C4, des halogènes de nombre atomique allant jusqu'à 35 inclus et/ou des groupes N,N-di-(aikyle inférieur)-amino contenant chacun 1 à 4 atomes de carbone, R₃ représente l'hydrogène ou un groupe alkyle inférieur en Cl-C4, n est égal à 0 et R₄ représente un groupe alkyle inférieur en Cl-C4, alcényle inférieur en C2-C4, phénylalkyle inférieur contenant de 1 à 4 atomes de carbone dans la partie alkyle, non substitué ou substitué par des groupes alkyles inférieurs en C1-C4, alcoxy inférieurs en C1-C4 et/ou des halogènes de nombre atomique allant jusqu'à 35 inclus, mono- ou di-hydroxyalkyles inférieurs en C2-C4 portant le ou les groupes hydroxy en position supérieure à la position alpha, (alcoxy inférieur)- ou (alkylthio inférieur)-alkyles inférieurs contenant 1 à 4 atomes de carbone dans la partie alcoxy ou alkylthio et 2 à 4 atomes de carbone dàns la partie alkyle, portant le groupe alcoxy ou alkylthio dans une position supérieure à la position alpha, et leurs sels formés par addition avec des acides.

6. Composés selon la revendication 1, dans lesquels l'un des symboles R₁ et R₂ représente un groupe pyridyle ou thiényle non substitué et l'autre un groupe phényle non substitué ou substitué par des groupes alkyles inférieurs en C1-C4, alcoxy inférieurs en C1-C4 et/ou des halogènes de nombre atomique allant jusqu'à 35 inclus, R₃ représente l'hydrogène ou un groupe alkyle inférieur en C1-C4, n est égal à 0, 1 ou 2 et R₄ représente un groupe alkyle inférieur en C1-C4, et leurs sels formés par addition avec des acides.

7. Composés selon la revendication 2, dans lesquels l'un des symboles R₁ et R₂ représente un groupe pyridyle non substitué et l'autre un groupe phényle non substitué ou substitué par des groupes alkyles inférieurs en C1-C4, alcoxy inférieurs en C1-C4 ou des halogènes, R₃ représente l'hydrogène ou un groupe alkyle inférieur en Cl-C4, n est égal à 0 et R₄ représente un groupe alkyle inférieur en C1-C4 ou un groupe hydroxy-alkyle inférieur en C2-C4 portant le groupe hydroxy dans une position supérieure à la position alpha, et leurs sels formés par addition avec des acides.

8. Composés selon la revendication 1, dans lesquels l'un des symboles R₁ et R₂ représente un groupe pyridyle et l'autre un groupe phénule, R₃ représente l'hydrogène, n est égal à 0 ou 1 et R₄ représente un groupe alkyle inférieur en Cl-C4, et leurs sels formés par addition avec des acides.

9. Composés selon l'une des revendications 1 à 8, en tant qu'agents anti-inflammatoires et/ou anti-nociceptifs.

10. Le 2-éthylthio-4(5)-phényl-5(4)-(3-pyridyl)-imidazole ou un sel de ce composé formé par addition avec un acide.

11. Le 2-éthane-sulfinyl-4(5)-phényl-5(4)-(3-pyridyl)-imidazole ou un sel de ce composé formé par addition avec un acide.

12. Le 2-éthylthio-4(5)-(p-fluorophényl)-5(4)-(2-thiényl)-imidazole.

13. Le 2-(2-hydroxyéthylthio)-4(5)-phényl-5(4)-(3-pyridyl)-imidazole ou un sel de ce composé formé par addition avec un acide.

14. Le 2-méthylthio-4(5)-phényl-5(4)-(3-pyridyl)-imidazole ou un sel de ce composé formé par addition avec un acide.

15. Le 2-(2-méthylthioéthylthio)-4(5)-phényl-5(4)-(3-pyridyl)-imidazole ou un sel de ce composé formé par addition avec un acide.

16. Le 2-(2-méthoxyéthylthio)-4(5)-phényl-5(4)-(3-pyridyl)-imidazole ou un sel de ce composé formé par addition avec un acide.

17. Le 2-propylthio-4(5)-phényl-5(4)-(3-pyridyl)-imidazole ou un sel de ce composé formé par addition avec un acide.

18. Le 2-isopropylthio-4(5)-phényl-5(4)-(3-pyridyl)-imidazole ou un sel de ce composé formé par addition avec un acide.

19. Le 2-éthane-sulfonyl-4(5)-phényl-5(4)-(3-pyridyl)-îmidazole ou un sel de ce composé formé par addition avec un acide.

20. Le 2-éthane-sulfinyl-4(5)-(p-fluorophényl)-(2-thiényl)-imidazole ou un sel de ce composé formé par addition avec un acide.

21. Le 2-éthane-sulfonyl-4(5)-(p-fluorophényi)-(2-thiényi)-imidazole ou un sel de ce composé formé par addition avec un acide.

22. Le 2-éthylthio-4,5-bis-(2-thiényl)-imidazole ou un sel de ce composé formé par addition avec un acide.

23. Composés de formule 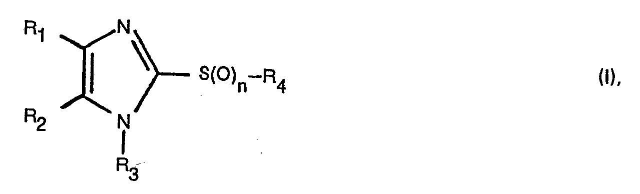 dans laquelle l'un au moins des symboles R₁ et R₂ représente un groupe hétéroaryle monocyclique, relié par l'intermédiaire d'un atome de carbone, à 5 chaînons contenant un atome d'oxygène ou de soufre, un atome de soufre et un atome d'azote ou au moins deux atomes d'azote, ou à 6 chaînons contenant un atome d'azote, non substitué ou substitué sur le carbone par des groupes alkyles inférieurs, alcoxy inférieurs, des atomes d'halogènes, des groupes amino, alkylamino inférieurs, di-(alkyle inférieur)-amino, alkylène- ou 3-aza-, 3-oxa- ou 3-thia-alkylène-amino contenant chacun 5 ou 6 chaînons cycliques et/ou trifluorométhyle et/ou substitué à l'azote par des groupes alkyles inférieurs, (alcoxy inférieur)-alkyles inférieurs, hydroxyalkyles inférieurs ou oxy, et un symbole R₁ ou R₂ différent du précédent représente un groupe phényle non substitué ou substitué par des groupes alkyles inférieurs, alcoxy inférieurs, des halogènes, des groupes amino, alkylamino inférieurs, di-(alkyle inférieur)-amino, alkylène-amino ou 3-aza-, 3-oxa- ou 3-thia-alkylène-amino contenant chacun 5 ou 6 chaînons cycliques, nitro et/ou trifluorométhyle, R₃ représente l'hydrogène ou un groupe alkyle inférieur, n est égal à 0, 1 ou 2 et R₄ représente un groupe alkyle inférieur, alcényle inférieur, ou alcynyle inférieur non substitué ou substitué par un groupe phényle non substitué ou substitué par des groupes alkyles inférieurs, alcoxy inférieurs, des halogènes, des groupes amino, alkylamino inférieurs, di-(alkyle inférieur)-amino, alkylène-amino ou 3-aza-, 3-oxa- ou 3-thia-alkylène-amino contenant chacun 5 ou 6 chaînons cycliques, nitro et/ou trifluorométhyle, ou un groupe alkyle inférieur substitué dans une position supérieure à la position alpha par des groupes hydroxy, alcoxy inférieurs, alkylthio inférieurs ou un groupe phénoxy ou-phényithio non substitué ou substitué par des groupes alkyles inférieurs, alcoxy inférieurs, des halogènes, des groupes amino, alkyl-amino inférieurs, di-(alkyle inférieur)-amino, alkylène-amino ou 3-aza-, 3-oxa- ou 3-thia-alkylène-amino contenant chacun 5 ou 6 chaînons cycliques, nitro et/ou trifluorométhyle, étant spécifié que dans les composés de formule dans laquelle n est égal à 1 ou 2, les groupes hétéroaryles R₁ et/ou R₂ diffèrent de groupes hétéroaryles à 6 chaînons portant un àtome de soufre ou d'oxygène et de groupes hétéroaryles à 6 chaînons portant un atome d'azote lorsque R₄ représente un groupe alkyle inférieur substitué par un ou deux groupes hydroxy, un groupe alcoxy inférieur ou un groupe phényle éventuellement substitué comme indiqué ci-dessus, ou un groupe alcynyle inférieur non substitué, l'expression "inférieur" s'appliquant à des restes contenant jusqu'à 7 atomes de carbone inclus, et leurs sels formés par addition avec des acides acceptables pour l'usage pharmaceutique, pour le traitement des maladies inflammatoires.

24. Composés de formule 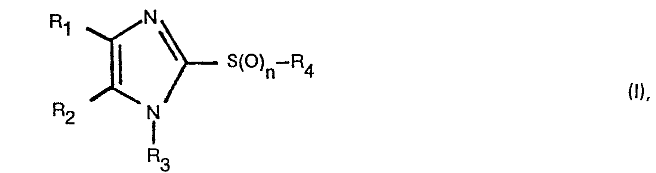 dans laquelle l'un an moins des symboles R₁ et R₂ représente un groupe hétéroaryle monocyclique, relié par l'intermédiaire d'un atome de carbone, à 5 chaînons contenant un atome d'oxygène ou de soufre, un atome de soufre et un atome d'azote, ou au moins deux atomes d'azote, ou à 6 chaînons contenant au moins un atome d'azote, non substitué ou substitué sur le carbone par des groupes alkyles inférieur, alcoxy inférieurs, des halogènes, des groupes amino, alkylamino inférieurs, di-(alkyle inférieur)-amino, alkylène- ou 5-aza, 3-oxa- ou 3-thia-alkylène-amino contenant chacun 5 ou 6 chaînons cycliques et/ou trifluorométhyle, et/ou substitué à l'azote par des groupes alkyles inférieurs, (alcoxy inférieur)-alkyles inférieurs ou hydroxyalkyles inférieurs, et un symbole R, ou R₂ différent du précédent représente un groupe phényle non substitué ou substitué par des groupes alkyles inférieurs, alcoxy inférieurs, des halogènes, des groupes amino, alkylamino inférieurs, di-(alkyle inférieur)-amino, alkylène-amino ou 3-aza-, 3-oxa- ou 3-thia-alkylène-amino contenant chacun 5 ou 6 chaînons cycliques, nitro et/ou trifluorométhyle, R₃ représente l'hydrogène ou un groupe alkyle inférieur, n est égal à O et R₄ représente un groupe alkyle inférieur, alcényle inférieur ou alcynyle inférieur non substitué ou substitué par un groupe phényle non substitué ou substitué par des groupes alkyles inférieurs, alcoxy inférieurs, des halogènes, des groupes amino, alkylamino inférieurs, di-(alkyle inférieur)-amino, alkylène-amino ou 3-aza-, 3-oxa- ou 3-thia-alkylène-amino contenant chacun 5 ou 6 chaînons cycliques, nitro et/ou trifluorométhyle, ou un groupe alkyle inférieur substitué dans une position supérieure à la position alpha par des groupes hydroxy, alcoxy inférieurs, alkylthio inférieurs, un groupe phénoxy ou phénylthio non substitué ou substitué par des groupes alkyles inférieurs, alcoxy inférieurs, des halogènes, des groupes amino, alkylamino inférieurs, di-(alkyle inférieur)-amino, alkylène-amino ou 3-aza-, 3-oxa- ou 3-thia-atkyiène-amino contenant chacun 5 ou 6 chaînons cycliques, nitro et/ou trifluorométhyle, l'expression "inférieur" s'appliquant à des restes contenant jusqu'à 7 atomes de carbone inclus, et leurs sels formés par addition avec des acides acceptables pour l'usage pharmaceutique, pour le traitement des maladies inflammatoires.

25. Composés selon l'une des revendications 2, 4, 5, 7, 10, 13 à 18 et 22 pour le traitement des maladies inflammatoires.

26. Composés selon l'une des revendications 1, 3, 6, 8, 9 et 19 à 21, pour le traitement des maladies inflammatoires.

27. Compositions pharmaceutiques contenant un composé selon l'une des revendications 2, 4, 5, 7, 10, 13 à 18, 22, 24 et 25 avec des produits auxiliaires pharmaceutiques usuels.

28. Compositions pharmaceutiques contenant un composé selon l'une des revendications 1, 3, 6, 8, 9, 19 à 21, 23 et 26 avec des produits auxiliaires pharmaceutiques usuels.

29. Procédé de préparation de composés de formule 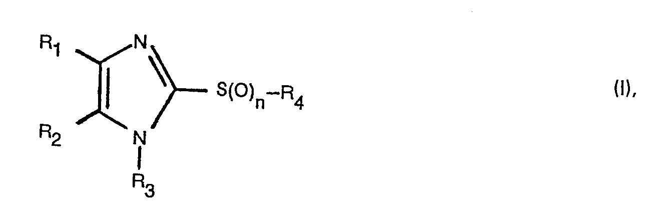 dans laquelle l'un au moins des symboles R₁ et R₂ représente un groupe hétéroaryle monocyclique, relié par l'intermédiaire d'un atome de carbone, à 5 chaînons contenant un atome d'oxygène ou de soufre, un atome de soufre et un atome d'azote ou au moins deux atomes d'azote, ou à 6 chaînons contenant au moins un atome d'azote, non substitué ou substitué sur le carbone par des groupes alkyles inférieurs, alcoxy inférieurs, des halogènes, des groups amino, alkylamino inférieurs, di-(alkyle inférieur)-amino, alkylène- ou 3-aza-, 3-oxa- ou 3-thia-alkylène-amino contenant chacun 5 ou 6 chaînons cycliques et/ou trifluorométhyle et/ou substitué à l'azote par des groupes alkyles inférieurs, (alcoxy inférieur)-alkyles inférieurs, hydroxyalkyles inférieurs ou oxy, et un symbole R, ou R₇ différent du précédent représente un groupe phényle non substitué ou substitué par des groupes alkyles inférieurs, alcoxy inférieurs, des halogènes, des groupes amino, alkylamino inférieurs, di-(alkyle inférieur)-amino, alkylène-amino ou 3-aza-, 3-oxa- ou 3-thia-alkylène-amino contenant chacun 5 ou 6 chaînons cycliques, nitro et/ou trifluorométhyle, R₃ représente l'hydrogène ou un groupe alkyle inférieur, n est égal à O, 1 ou 2 et R₄ représente un groupe alkyle inférieur, alcényle inférieur ou alcynyle inférieur non substitué ou substitué par un groupe phényle non substitué ou substitué par des groupes alkyles inférieurs, alcoxy inférieurs, des halogènes, des groupes amino, alkylamino inférieurs, di-(alkyle inférieur)-amino, alkylène-amino ou 3-aza-, 3-oxa- ou 3-thia-alkylène-amino contenant chacun 5 ou 6 chaînons cycliques, nitro et/ou trifluorométhyle, ou un groupe alkyle inférieur substitué dans une position supérieure à la position alpha par des groupes hydroxy, alcoxy inférieurs, alkylthio inférieurs ou un groupe phénoxy ou phénylthio non substitué ou substitué par des groupes alkyles inférieurs, alcoxy inférieurs, des halogènes, des groupes amino, alkylamino inférieurs, di-(alkyle inférieur)-amino, alkylène- amino ou 3-aza-, 3-oxa- ou 3-thia-alkylène-amino contenant chacun 5 ou 6 chaînons cycliques, nitro et/ou trifluorométhyle, et leurs sels formés par addition avec des acides, l'expression "inférieur" s'appliquant à des restes qui contiennent jusqu'à 7 atomes de carbone inclus, caractérisé en ce que:
a) on fait réagir entre eux des composés de formules 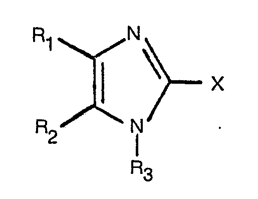 (II) et Y‾R₄ (III), dans lesquelles l'un des symboles X et Y est un groupe mercapto éventuellement à l'état de sel et l'autre un reste échangeable contre un groupe mercapto éthérifié, étant spécifié que dans les composés de formule Il dans laquelle X représente un groupe mercapto éventuellement à l'état de sel, les groupes hétéroaryles R, et/ou R₂ diffèrent d'un groupe hétéroaryle à 5 chaînons contenant un atome de soufre et d'un groupe hétéroaryle à 6 chaînons contenant un atome d'azote ou bien les restes R₃ diffèrent de l'hydrogène lorsque, dans les composés de formule Ili, Y représente un groupe hydroxy estérifié réactif et R₄ un groupe alkyle inférieur monosubstitué par un groupe hydroxy en position 2, 3 ou 4, ou bien
b) on fait réagir entre eux des composés de formules 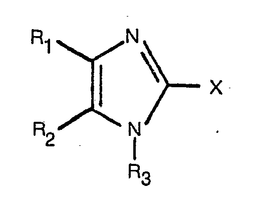 (II) et Y-R₄ (III), dans lesquelles l'un des symboles X et Y représente un groupe de formule ‾S(O)ₙY₁ dans laquelle Y₁ représente un groupe hydroxy estérifié réactif ou bien, lorsque n est égal à O, un groupe mercapto éthérifié ou bien, lorsque n est égal à 1 ou 2, un groupe hydroxy éthérifié, et l'autre représente un radical métallique Y₂, ou bien
c) on fait réagir un composé de formule 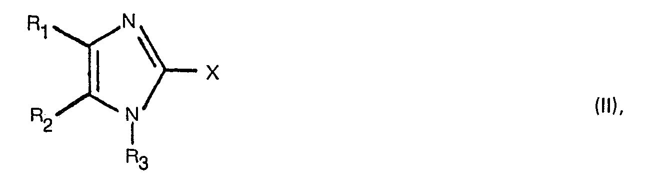 dans laquelle X représente le groupe mercapto, avec un alcène inférieur éventuellement substitué comme indiqué pour le groupe alkyle inférieur R₄, ou bien
d) on fait réagir un composé de formule 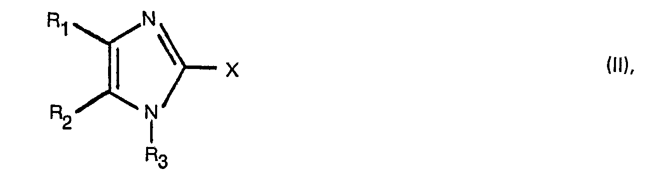 dans laquelle X représente un groupe acide sulfinique ou acide sulfonique à l'état de sel, avec un composé de formule 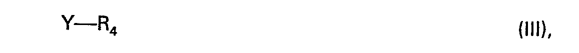 dans laquelle Y représente un groupe hydroxy estérifié réactif, ou bien
e) on fait réagir un composé de formule 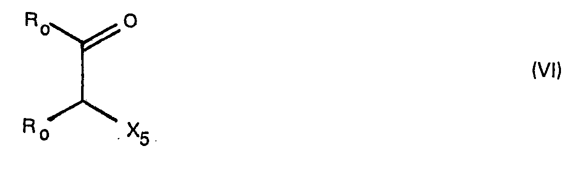 dans laquelle l'un des symboles Ro représente un groupe R₁ et l'autre un groupe R₂ et X₅ représente un groupe hydroxy estérifié réactif, ou un sel d'un tel composé formé par addition avec un acide, avec une N‾R₃‾S‾R₄‾isothiourée, ou bien
f) on transpose un composé de formule 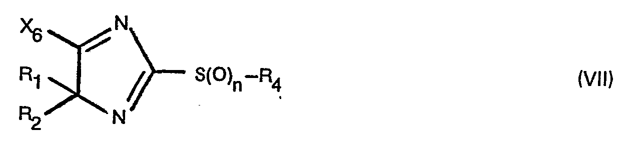 dans laquelle X₆ est un groupe alkylthio inférieur éventuellement substitué par un groupe phényle qui peut lui-même être substitué comme R₁ et R₂, ou un sel d'un tel composé formé par addition avec un acide, et si on le désire, dans un composé de formule 1 dans laquelle n est égal à 0 et R₁, R_{2'} R₃ et R₄ ont les significations indiquées ci-dessus, obtenu selon les variantes opératoires a) à f), étant spécifié que dans les composés de formule 1 obtenus selon la variante opératoire a) par réaction des composés de formules II et III dans lesquelles X représente un groupe mercapto éventuellement à l'état de sel et Y un groupe hydroxy estérifié réactif, les groupes hétéroaryles, R₁ et/ou R₂ diffèrent de groupes hétéroaryles à 5 chaînons portant un atome de soufre ou d'oxygène ou de groupes hétéroaryles à 6 chaînons portant un atome d'azote lorsque R₄ représente un groupe alkyle inférieur substitué par un ou deux groupes hydroxy, un groupe alcoxy inférieur, ou par un groupe phényle éventuellement substitué comme indiqué ci-dessus, ou un groupe alcynyle inférieur non substitué, on oxyde le groupe thio en groupe sulfinyle ou sulfonyle et/ou dans les composés obtenus selon l'une des variantes opératoires a) à f), dans lesquels R₁ et/ou R₂ porte au moins un atome d'azote ⋟N, on oxyde à l'azote, ou dans des composés obtenus selon l'une des variantes opératoires b) ou f) et dans lesquels n est égal à 1 ou 2, on réduit le groupe sulfinyle ou sulfonyle en groupe thio et/ou dans les composés obtenus selon les variations opératoires a) à f) et dans lesquels R₁ et R₂ contiennent un atome d'azote oxydé, on réduit cet atome d'azote en un groupe ⋟N, on alkyle à l'azote par un groupe alkyle inférieur un composé de formule 1 obtenu conformément à l'invention dans lequel R₃ représente l'hydrogène, dans les composés de formule I obtenus conformément à l'invention, dans les restes R₁, R₂ et/ou les groupes phényle, phénoxy ou phénylthio constituants de R₄, on introduit éventuellement des substituants additionnels sur le carbone et/ou on convertit un composé obtenu conformément à l'invention à l'état libre en un sel formé par addition avec un acide ou un sel d'acide obtenu conformément à l'invention en le composé libre ou en un autre sel.

30. Procédé selon la revendication 29, pour la préparation des composés de formule I dans laquelle R₁, R₂, R₃ et R₄ ont les significations indiquées dans la revendication 29, les groupes hétéroaryles R₁ et/ou R₂ ne portant pas toutefois de substituant oxy à l'azote, et n est égal à 0, et de leurs sels formés par addition avec des acides, caractérisé en ce que:
a) on fait réagir entre eux des composés de formules 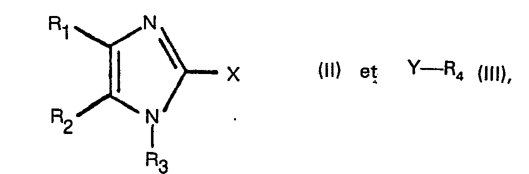 dans lesquelles l'un des symboles X et Y représente un groupe mercapto éventuellement à l'état de sels et l'autre un reste échangeable contre un groupe mercapto éthérifié, étant spécifié que dans les composés de formule Il dans laquelle X représente un groupe mercapto éventuellement à l'état de sel, les groupes hétéroaryles R₁ et/ou R₂ diffèrent d'un groupe hétéroaryle à 5 chaînons contenant un atome de soufre ou d'un groupe hétéroaryle à 6 chaînons portant un atome d'azote ou bien les restes R₃ diffèrent de l'hydrogène lorsque, dans les composés de formule III, Y représente un groupe hydroxy estérifié réactif et R₄ un groupe alkyle inférieur monosubstitué par un groupe hydroxy en position 2, 3 ou 4, ou bien
c) on fait réagir un composé de formule 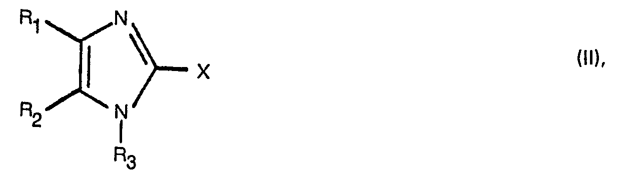 dans laquelle X représente un groupe mercapto, avec un alcène inférieur éventuellement substitué comme indiqué pour le groupe alkyle inférieur R₄, ou bien
e) on fait réagir un composé de formule 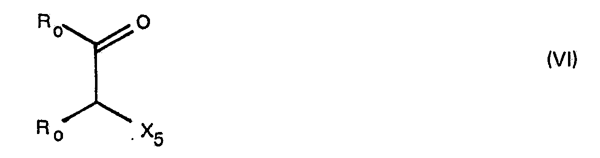 dans laquelle le reste Ro en position alpha du groupe oxo est un reste R₁ et l'autre reste Rₒ est un reste R₂, et X₅ représente un groupe hydroxy estérifié réactif, avec un sel de N‾R₃‾S‾R₄-isothiuronium et si on le désire on alkyle à l'azote par un groupe alkyle inférieur un composé de formule I obtenu conformément à l'invention et dans lequel R₃ représente l'hydrogène, dans les composés de formule obtenus conformément à l'invention, dans les restes R₁, R₂ et/ou les groupes phényle, phénoxy ou phénylthio constituants de R₄, on introduit éventuellement des substituants additionnels au carbone, et/ou on convertit un composé obtenu conformément à l'invention à l'état libre en un sel formé par addition avec un acide ou un sel d'acide obtenu conformément à l'invention en le composé libre.

31. Les composés obtenus par le procédé de la revendication 29 ou 30.

32. Composés selon l'une des revendications 1 à 26 en tant qu'agents anti-inflammatoires.

33. Utilisation des composés selon l'une des revendications 1 à 26 pour la préparation de médicaments par voie non. chimique.
